(19) Europäisches Patentamt
European Patent Office
Office européen des brevets

(11) **EP 3 171 944 B1**

(12) **EUROPÄISCHE PATENTSCHRIFT**

(45) Veröffentlichungstag und Bekanntmachung des Hinweises auf die Patenterteilung:
**28.10.2020 Patentblatt 2020/44**

(21) Anmeldenummer: **15738700.2**

(22) Anmeldetag: **21.07.2015**

(51) Int Cl.:
*A61Q 15/00* [(2006.01)] *A61Q 17/00* [(2006.01)]
*A61K 8/81* [(2006.01)]

(86) Internationale Anmeldenummer:
**PCT/EP2015/066593**

(87) Internationale Veröffentlichungsnummer:
**WO 2016/012417 (28.01.2016 Gazette 2016/04)**

(54) **VERWENDUNG VON POLYQUATERNIUM POLYMERE IN DEODORANTZUBEREITUNGEN**

USE OF POLYQUATERNIUM POLYMERS IN DEODORANT COMPOSITIONS

UTILISATION DE POLYMÈRES POLYQUATERNIUM DANS DES COMPOSITIONS DÉODORANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.07.2014 DE 102014214463**

(43) Veröffentlichungstag der Anmeldung:
**31.05.2017 Patentblatt 2017/22**

(73) Patentinhaber: **Beiersdorf AG**
**20253 Hamburg (DE)**

(72) Erfinder:
• **TRAUPE, Bernd**
  **24568 Kaltenkirchen (DE)**
• **WEETS, Gudrun**
  **22527 Hamburg (DE)**
• **GROTH, Inken**
  **20257 Hamburg (DE)**
• **FIRYN, Andreas**
  **21493 Schwarzenbek (DE)**
• **KLAWITER, Helen**
  **20255 Hamburg (DE)**
• **KLAUCK, Robert**
  **21465 Reinbek (DE)**
• **PRIEBE, Laura**
  **22049 Hamburg (DE)**
• **BRAREN, Sandra**
  **20251 Hamburg (DE)**
• **FÖLSTER, Heike**
  **22149 Hamburg (DE)**
• **KANINCK, Stefanie**
  **22359 Hamburg (DE)**
• **LIEBICH, Anne**
  **22085 Hamburg (DE)**
• **SEET, Michael**
  **20253 Hamburg (DE)**

(56) Entgegenhaltungen:
EP-A2- 0 200 548    WO-A1-00/06104
WO-A1-2005/087188   DE-A1-102010 007 958
JP-A- 2012 177 901  JP-A- 2014 101 356
US-A- 4 478 821     US-A1- 2007 267 035
US-A1- 2009 004 130 US-A1- 2012 009 138

• "International Cosmetic Ingredient Dictionary and Handbook", 2012, Personal Care Products Council, XP002743795, Bd. 2, Seite 2565,2568, Einträge "POLYQUATERNIUM-6" und "POLYQUATERNIUM-16"
• Anonymous: "Deodorant - Wikipedia", , 4. Juli 2015 (2015-07-04), Seiten 1-4, XP55208564, Gefunden im Internet: URL:https://de.wikipedia.org/wiki/Deodoran t [gefunden am 2015-08-19]
• DATABASE GNPD [Online] MINTEL; September 2013 (2013-09), "Perfect Curls Mousse Gel", XP002751272, Database accession no. 2192259

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**EP 3 171 944 B1**

**Beschreibung**

Die Erfindung ist die Verwendung von Polyquaternium- Polymeren ausgewählt aus der Gruppe Polyquaternium-16-, Polyquaternium-6- und/oder Polyquaternium-37 Polymere als antimikrobieller Deodorantwirkstoff in kosmetischen oder dermatologischen Zubereitungen.

Antitranspirantien (AT) oder Desodorantien (Deo) dienen dazu, Körpergeruch zu beseitigen oder zu verhindern, der entsteht, wenn der an sich geruchlose frische Schweiß durch Mikroorganismen zersetzt wird. Den üblichen kosmetischen Desodorantien liegen unterschiedliche Wirkprinzipien zugrunde.

Im allgemeinen Sprachgebrauch erfolgt nicht immer eine klare Trennung der Begriffe "Deodorant/Desodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die, im Gegensatz zu den Desodorantien, die Absonderung von Schweiß überhaupt verhindern sollen. Desodorantien verhindern im Gegensatz dazu im Allgemeinen eine mikrobielle Zersetzung von bereits gebildetem Schweiß.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Schweißgeruch besteht zu einem Großteil aus verzweigtkettigen Fettsäuren, die durch bakterielle Enzyme aus geruchlosem Schweiß freigesetzt werden. Klassische Deo-Wirkstoffe wirken dem entgegen, indem sie das Wachstum von Bakterien reduzieren.

Entscheidend für eine desodorierende Wirkung ist es dabei, wenn desodoriende Wirkstoffe vornehmlich gegen die Bakterien Staphylococcus epidermidis und Corynebacterium jeikeium wirken.

Polyquaternium Polymere sind nach der INCI-Nomenklatur Bezeichnungen für komplexe quartäre Ammonium-Polymerverbindungen. Die verschiedenen Vertreter dieser Gruppe werden durch Ziffern unterschieden. Zum Beispiel Polyquaternium-1, Polyquaternium-16 (PQ-16), Polyquaternium-42, usw. Als quartäre Ammoniumverbindungen besitzen Polyquaternium-Polymere quartäre Stickstoffatome, d. h. alle vier Wasserstoffatome des Ammonium-Ions sind durch organische Reste ersetzt. Die Verbindungen sind somit kationisch, besitzen also eine positive Ladung.

In der WO 2013052454 A1 ist eine Übersicht bekannter Polyquaternium Polymere und deren Handelsnamen aufgeführt.

Vertreter dieser nach INCI benannten Gruppe sind u. a. wegen ihrer filmbildenden und antistatischen Eigenschaften in vielen Körperpflegeprodukten enthalten, z. B. in Shampoos.

Polyquaternium-6 Polymer (PQ-6) ist ein polymeres quaternäres Ammoniumsalz gebildet aus der Homopolymerisation des Diallyldimethylammoniumchlorid (DADMAC) Monomers. Polyquaternium-16 Polymere sind 3-Methyl-1-Vinylimidazolium Chloride-1-Vinyl-2-Pyrrolidinone Chloride, wie sie unter den Handelsnamen Luviquat® angeboten werden, wie in nachfolgender Tabelle 1 dargestellt:

| Handelsname | Feststoffgehalt % | Molmasse | Verhältnis VP/QVI | Ladungsdichte (meq/g) |
|---|---|---|---|---|
| Luviquat Excellence | 38-42 | 40,000 | 5/95 | 6,1 |
| Luviquat FC 550 | 38-42 | 80,000 | 50/50 | 2,0 |
| Luviquat FC 370 | 38-42 | 100,000 | 70/30 | 3,3 |
| Luviquat Style | 19-21 | 400,000 | 55/45 | 3,0 |

Als PQ-6 Polymer ist bekannt

| Handelsname | Feststoffgehalt % | Ladungsdichte (meq/g) |
|---|---|---|
| Mirapol 100 | 39-41 | 6,2 |

In der EP 1504744 B1 werden Polyquaternium-16 Polymere im Bereich der Haarpflege, insbesondere in Haarsprays, angewendet.

In der US 20070184016 A1 und WO 2007095008 A2 werden Polyquaternium-16 Polymere in Desinfektionsmitteln beschrieben, wie auch alkoholische Lösungen. Diese umfassen jedoch einen Alkoholgehalt von mindestens 50%. Aufgrund der bekannten hohen kationischen Ladungsdichte der Polymere ist zur homogenen Stabilisierung im Produkt der Einsatz einer großen Menge an Ethanol notwendig.

In der EP 1991654 A1 werden Polyquaternium-16 Polymere, z.B. unter dem Namen Luviquat FC 905 erhältlich, in Seifenstückmasse beschrieben.

Die EP 200548 A2 beschreibt Deodorantzubereitungen umfassend antimikrobielle Wirkstoffe, wie Triclosan oder Benzalkoniumchlorid. Die Zubereitungen umfassen des Weiteren kationische Polymere, insbesondere Copolymere der Dimethyldiallyammoniumchloride (DMDAAC), wie Merquat S® oder Merquat 550® (PQ-7).

Diese kationischen Polymere verbessern die antimikrobielle Wirksamkeit der antimikrobiellen Wirkstoffe aufgrund ihrer Substantivität und Filmbildungseigenschaften. D.h. sie sorgen dafür, dass die antimikrobiellen Wirkstoffe länger am Wirkort verbleiben können und somit erst eine verbesserte Wirksamkeit zeigen können. Ein Hinweis darauf, dass Polyquaternium Polymere per se eine antimikrobielle Wirksamkeit und ggf. eine desodorierende Wirkung aufweisen, ist nicht gegeben.

Schweißhemmung aufgrund der Bildung okklusiver Filme wird in der WO 1995/24105 A1 und Schweißhemmung aufgrund adstringierender und flüssigkeitsabsorbierender Wirkungen wird in der US 4743440 und WO 2003/030853 A2 beschrieben.

In der WO 2009091794 A1 werden Zubereitungen beschrieben, die neben antitranspirant wirksamen Mitteln kationische Polyquaternium Polymere, PQ-6, PQ-7, PQ-10, PQ-11, PQ-22 und insbesondere PQ-28, umfassen.

In der EP 1761241 A1 werden antitranspirant wirksame Stoffe auf Basis von Diallydimethylammoniumchloriden (DADMAC) und Diallyethylammoniumchloriden (DADEAC) beschrieben, insbesondere PQ-6 (Merquat® 100, Flocare® C106, Floquat FL45®), Polyquaternium-33 (Floerger FO 4190 VHM®,4550 BPM®), Polyquaternium-5 (Merquat 5®, Reten®) und Polyquaternium-7 (Merquat 550 L®, Salcare Super 7®, Flocare C107®).

Ein Hinweis auf eine desodorierende Wirksamkeit dieser Stoffe ist damit nicht gegeben, da es einen eklatanten Unterschied im Wirkmechanismus zwischen Schweißhemmung (antitranspirant wirksam) und Geruchsverbesserungsmittel (desodorierend) gibt.

In der JP 2014101356 wird die Kombination von Polyquaternium-10 mit antimikrobiellen Stoffen, wie Benzalkoniumchlorid oder Isoropylmethylphenol, als Waschmittel beschrieben, die eine Körpergeruchsverbesserung, insbesondere für ältere Menschen, hervorrufen soll.

In den Vergleichstests wird eine Zubereitung mit Polyquaternium-10 und ohne antimikrobiellem Mittel als nicht geruchsverbessernd angesehen.

In der Zeitschrift für Fett-, Öl-, Kosmetik-, Pharma- und Waschmittelindustrie, 111. Jahrgang, Nr. 4, vom 6. März 1985 werden verschiedene polyquaternäre Polymere mit abgestufter Kationenaktivität vorgestellt, die unter der Handelsbezeichnung Luviquat 905, 550 und 370 der BASF erhältlich sind. Die Polymere unterscheiden sich durch die Kombination mit Vinylpyrrolidon (VP) und quaternisiertem Vinylimidazol (VI, QVI). Ihr Einsatz ist für Haarzubereitungen beschrieben.

Probleme ergeben sich beim Einsatz von Polyquaternium Polymeren, insbesondere PQ-6 und PQ-16, aufgrund ihrer hohen kationischen Ladungsdichte, wodurch es in den Zubereitungen zu Ausfällungen, Agglomerationen und Phasenbildungen kommen kann.

Wünschenswert ist es daher homogene, stabile Formulierungen, die Polyquaternium Polymere umfassen und die auch unter thermischer Belastung ihr Erscheinungsbild nicht verändern, zur Verfügung zu stellen.

Wünschenswert ist es darüber hinaus O/W-Emulsionen, unterscheidbar in Mikro-und Makroemulsionen, umfassend PQ Polymere, insbesondere PQ-16 Polymere, bereit zu stellen.

Im Stand der Technik sind kosmetische Zubereitungen, beispielsweise als Deodorantien oder Antitranspirantien, beschrieben, die ein oder mehrere Polyquaternium Polymere umfassen. Damit ist jedoch noch nicht offenbart, dass die Polyquaternium Polymere per se als Deodorantwirkstoff agieren.

Die Anwendungsformen und Arten von Antitranspirantien und Deodorantien unterliegen häufig dem Zeitgeschmack. In einer mehr und mehr hektischen Umgebung sollte das Pflegen, wie das Deosodorieren, möglichst wenig Zeit in Anspruch nehmen. Des Weiteren wird zunehmend eine "Zwischendurch-Anwendung" von Deodorant- oder Antitranspirantmittel gewünscht.

Wünschenswert ist es kosmetische Zubereitungen als Desodorant I zu verwenden, die ohne die geschilderten Nachteile des Standes der Technik angewendet werden können. Insbesondere ist es wünschenswert kosmetische Desodorantien zu verwenden, die auch auf der Kleidung angewendet werden können und dennoch kosmetisch wirksam sind.

Die Erfindung ist die Verwendung von Polyquaternium Polymeren ausgewählt aus der Gruppe Polyquaternium-16-, Polyquaternium-6- und/oder Polyquaternium-37 Polymere als antimikrobieller Deodorantwirkstoff in kosmetischen oder dermatologischen Zubereitungen.

Bevorzugt werden Polyquaternium-16 und/oder Polyquaternium-6 Polymere als Deodorantwirkstoff verwendet.

Polyquaternium Polymere sind als Filmbildner insbesondere in Haarzubereitungen bekannt. Zu erwarten ist daher, dass kosmetische Zubereitungen umfassend Polyquaternium Polymere sich auf der Haut und Haaren fest anlagern bzw. aufziehen.

Erstaunlicherweise lassen sich die verwendeten Zubereitungen umfassend ein oder mehrere Polyquaternium Polymere, insbesondere PQ-16 und/oder Polyquaternium-6, und insbesondere als Mikroemulsion formuliert besonders leicht von der Haut wieder entfernen. Sie lassen sich erstaunlicherweise auch leicht wieder abwaschen.

Aus der Färbetechnologie von Textilien ist bekannt, dass kationische Farbstoffe auf natürlichen Fasern nur mäßig waschechte Farben bilden, da sie nur mäßig gut auf den Fasern binden.

Erstaunlicherweise binden jedoch diese Polyquaternium Polymere, insbesondere PQ-16 und PQ-6, obwohl sie kationische Ladungen tragen, sehr gut auf Textilfasern. In Untersuchungen zur desodorierenden Wirksamkeit konnte gezeigt werden, dass diese Polyquaternium Polymere aufgebracht auf Textilien zu einer signifikanten Geruchsverbesserung führen.

Was dazu führt, dass die verwendeten Zubereitungen auch auf Textilien aufziehen können und auch dort den Schlechtgeruch bekämpfen können.

Die Verwendung von ein oder mehreren Polyquaternium Polymeren ausgewählt aus der Gruppe Polyquaternium-16, Polyquaternium-6 und/oder Polyquaternium-37, insbesondere Polyquaternium-16 und/oder PQ-6, zur Desodorierung auf oder durch die Kleidung ist damit möglich.

Mittels der verwendeten Zubereitung kann das Kosmetikum auf die Haut appliziert werden, wobei vorteilhaft dies direkt durch die Kleidung erfolgen kann.

Als geeignete Kleidung sind in diesem Zusammenhang selbstverständlich diejenigen Kleidungsformen, die aufgrund ihrer Materialien oder deren Herstellung keinerlei Substanz aufnehmen oder hindurch lassen, ausgenommen.

Die Polyquaternium Polymere, ausgewählt aus der Gruppe Polyquaternium-16, Polyquaternium-6 und/oder Polyquaternium-37, insbesondere PQ-16 und PQ-6, zeigen eine desodorierende Wirksamkeit. Im Gegensatz zu den Antitranspirantien bewirken die Desodorantien keine aktive Beeinflussung der Schweißsekretion, sondern lediglich die Steuerung bzw. Beeinflussung des Körper- bzw. Achselgeruches (Geruchsverbesserungsmittel). In diesem Sinne werden die erfindungsgemäßen Polyquaternium Polymere, insbesondere Polyquaternium-16 Polymere (PQ-16) und Polyquaternium-6 (PQ-6), als Deodorantien verwendet.

Die Zubereitungen können dabei ein oder mehrere weiteren desodorierend und/oder antitranspirant wirkende Stoffe umfassen. Bevorzugt ist es aber, dass die verwendeten Zubereitungen insbesondere keine weiteren antitranspirant wirkende Stoffe umfassen, insbesondere keine Aluminiumsalze.

Des Weiteren sind die verwendeten Zubereitungen bevorzugt frei von organischen Verbindungen mit anionischer Ladung. Die klassischen Gegenanionen, wie Chloridionen oder Bromidionen, sind nicht als organische Verbindungen mit negativer Ladung zu verstehen. Bevorzugt wird auf die Anwesenheit von lipophilen organischen Verbindungen, insbesondere Polymeren mit anionischer Ladung verzichtet, da es ansonsten zu Agglomeration, Ausfall und Instabilitäten kommen kann. Als Verbindungen mit anionischer Ladung werden Verbindungen verstanden, die eine negativ geladene funktionelle Gruppe besitzen.

Des Weiteren ist der Zusatz an weiteren antimikrobiellen Stoffen nicht zwingend erforderlich. Hinsichtlich der Lagerstabilität können selbstverständlich Konservierungsverbesserer zugesetzt werden, wie beispielsweise Octopirox oder Phenoxyethanol.

Da die Polyquaternium PQ-16, PQ-6 und PQ-37, insbesondere PQ-16 und PQ-6, desodorierende Wirkungen aufweisen, kann auch auf den Zusatz anderer Deodorantwirkstoffe bevorzugt verzichtet werden.

Obwohl Parfumstoffe auch als Geruchsverbesserer gelten, sind Parfumstoffe hierbei explizit ausgenommen und die verwendeten Zubereitungen können Parfum mit umfassen.

Aufgrund der Stabilitätsproblematik von Zubereitungen umfassend Polyquaternium Polymeren mit hoher kationischer Ladungsdichte kann die Stabilisierung PQ-haltiger Zubereitungen wie nachstehend ausgeführt gelöst werden.

Die verwendete kosmetische oder dermatologische Zubereitung umfasst a.) ein oder mehrere Polyquaternium Polymere und ein oder mehrere Stoffe gewählt aus den Gruppen b.) nichtionischen Emulgatoren, c.) kationischen Emulgatoren und d.) Rheologiemodifizierer.

Die Stoffgruppe b.) umfasst ein oder mehrere nichtionische Emulgatoren. Der Gesamt HLB Wert der nichtionischen Emulgatoren, eines einziges nichtionischen Emulgators oder einer Mischung zweier oder mehrerer nichtionischer Emulgatoren, liegt dabei bevorzugt im Bereich von größer 9 bis zu 17. Ist die Zubereitung auf Basis einer Makroemulsion formuliert liegt der Gesamt HLB-Wert der Emulgatormischung idealerweise zwischen 3 bis 14, insbesondere im Bereich 4 bis 12, vorzugsweise im Bereich 7 bis 10..

D.h. es kann einerseits ein einziger nichtionischer Emulgator mit einem HLB Wert größer 9 und bis zu 16 bzw. 3 bis 14 gewählt werden (z.B. Laureth-4 - HLB 9,7) oder mehrere nichtionische Emulgatoren mit unterschiedlichen HLB - Werten, wobei der HLB - Wert der Mischung dann in dem erfindungsgemäßen Bereich liegen sollte (z.B. Glyceryl Isostearate HLB 3,5 und Isoceteth-20 (HLB 15.7)). Dabei spielt auch deren jeweiliger Anteil und das Verhältnis zueinander eine erfindungsgemäße Rolle, wie nachfolgend ausgeführt wird.

Die Stoffgruppe c.) umfasst ein oder mehrere kationische Emulgatoren und die Stoffgruppe d.) ein oder mehrere Rheologiemodifizierer.

Rheologiemodifizierer (d.) sind Stoffe, die die Viskosität der sie enthaltenen Zubereitung beeinflussen, insbesondere erhöhen.

Bevorzugt umfassen die verwendeten Zubereitung mindestens zwei unterschiedliche Polyquaternium Polymere, insbesondere gewählt aus der Gruppe der Polyquaternium-16 Polymeren und Polyquaternium-6 Polymeren. D.h. eine Zubereitung umfassend Polyquaternium-16 und Polyquaternium-6 Polymere sind bevorzugt zu wählende Deoformulierungen.

Die verwendeten Zubereitungen sind bevorzugt frei von organischen Verbindungen mit anionischer Ladung.

Frei von bedeutet dabei, dass der Anteil an diesen Stoffen auf die verzichtet werden kann, wie anionische Stoffe, antitranpirant wirksame Stoffe oder zusätzliche Deodorantien, weniger als 0,1 Gew.%, bezogen auf die Gesamtmasse der Zubereitung, beträgt. Dadurch wird gewährleistet, dass Einschleppungen oder Verunreinigungen mit diesen Stoffen nicht als erfindungsgemäß "frei von" mitumfasst wird.

Bevorzugt werden die Polyquaternium Polymere a.) ausgewählt aus der Gruppe der Polyquaternium-16 Polymere (3-Methyl-1-Vinylimidazolium Chlorid-1-Vinyl-2-Pyrrolidinon Chloride), die

- gebildet sind aus einem Monomerverhältnis von Vinylpyrrolidon (VP) zu quaternisiertem Vinylimidazol (QVI) im Bereich von 1 bis 99% (VP) zu 99 bis 1% (QVI),
- eine Molmasse im Bereich von 10.000 bis 1.000.000 Da und
- eine Ladungsdichte im Bereich von 1 bis 7 meq/g aufweisen.

Weiterhin erfindungsgemäß bevorzugt ist die Verwendung von ein oder mehreren Polyquaternium-16 Polymere (3-Methyl-1-Vinylimidazolium Chlorid-1-Vinyl-2-Pyrrolidinon Chloride), die

- gebildet sind aus einem Monomerverhältnis von Vinylpyrrolidon (VP) zu quaternisiertem Vinylimidazol (QVI) im Bereich von 1 bis 99% (VP) zu 99 bis 1% (QVI),
- eine Molmasse im Bereich von 10.000 bis 1.000.000 Da und
- eine Ladungsdichte im Bereich von 1 bis 7 meq/g aufweisen

als antimikrobieller Deodorantwirkstoff.

Als erfindungsgemäß besonders bevorzugt haben sich Polyquaternium-16 Verbindungen (PQ-16) herausgestellt, die durch folgende Parameter gekennzeichnet sind:

- Monomerverhältnis VP/QVI (Gew.%): 1-99 / 99-1, bevorzugt 4 - 8 / 92 - 96, insbesondere 7/93 bis 5/95, insbesondere im Bereich von 5 bis 95% (VP/QVI),
- Ladungsdichte (meq/g): 4 bis 7, insbesondere 5 bis 6,5, bevorzugt zwischen 5,8n und 6,2
- Molmasse (Da): 10.000 - 1.000.000, bevorzugt 20.000 - 80.000.

Die Handelsbezeichnungen Luviquat® umfassen erfindungsgemäß bevorzugten PQ-16 Polymere, wie in Tabelle 1 angegeben. Der Aktivgehalt an PQ-16 Polymeren (Feststoffanteil) liegt dabei im Bereich von 19 bis 42 Gew.%. Der Rest besteht aus Wasser.

Der Anteil an einem oder mehreren Polyquaternium Polymeren in kosmetischen oder dermatologischen Zubereitungen beträgt vorteilhaft insgesamt bis zu 10 Gew.%, bevorzugt bis zu 2 Gew.% und minimal 0,01 Gew.%, jeweils bezogen auf die Gesamtmasse der Zubereitung.

Der Anteil an einem oder mehreren nichtionischen Emulagtoren (b.), kationischen Emulgatoren (c.) und /oder Rheologiemodifizierer (d.) liegt jeweils vorzugsweise im Bereich bis zu 10 Gew.%, insbesondere im Bereich von 0,1 bis 8 Gew.%, bezogen auf die Gesamtmasse der Zubereitung.

Bevorzugt sind die verwendeten Zubereitungen als Mikroemulsion, wässrig-alkoholischen Formulierungen oder Makroemulsionen formuliert.

Erfindungsgemäße Polyquaternium Polymere Polyquaternium-16, Polyquaternium-6 und/ oder Polyquaternium-37 und insbesondere die PQ-16 und PQ-6 Polymere zeigen eine gute Deowirksamkeit, wie in nachfolgenden Untersuchungen gezeigt wird.

Die Deowirksamkeit ist dabei für die bevorzugt einzusetzenden Polyquaternium Polymere PQ-16 und PQ-6 als auch für PQ-37 gezeigt worden. Es ist damit generell eine Deowirksamkeit von Polyquaternium Polymeren erstmalig gezeigt worden.

Der Einsatz von Polyquaternium Polymeren Polyquaternium-16, Polyquaternium-6 und/oder Polyquaternium-37 und insbesondere PQ-16 und/oder PQ-6 als Deodorantwirkstoff in kosmetischen Deodorantzubereitungen ist damit möglich und erfindungsgemäß.

Aufgrund der relativ hohen kationischen Ladungsdichte der PQ-16 Verbindungen ist eine Einarbeitung in kosmetischen Formulierungen problematisch, da es zu Ausfällungen und/oder Agglomerationen von Emulgator- bzw. Verdickersystemen der Zubereitungen oder es sogar zur Inaktivierung der Wirkstoffe kommen kann.

Besonders bei opaken Formulierungen, beispielsweise opaken Makroemulsionen, kann die Stabilität durch die Anwesenheit von Polyquaternium Polymeren stark negativ beeinflusst werden.

Die Stabilisierung kosmetischer Zubereitungen umfassend Polyquaternium Verbindungen, insbesondere erfindungsgemäße PQ-16 Verbindungen, insbesondere in Mikroemulsionen, dabei vornehmlich Mikroemulsionsgele, und bevorzugt wässrig-alkoholische Formulierungen und Makroemulsionen, erfolgt indem diesen Zubereitungen die Emulgatoren (nichtionische Emulgatoren b.) und/oder kationische Emulgatoren c.)) und/oder Rheologiemodifizierer (d.), welche keine negative Ladung tragen, zugesetzt werden. D.h. es werden kationische und/oder nichtionische Emulgatoren und/oder kationische und/oder Rheologiemodifizierer, bevorzugt nichtionische Modifizierer, bevorzugt zur Stabilisierung eingesetzt.

Bevorzugt ist eine Makroemulsion, die Emulgatoren und/oder Rheologiemodifizierer, welche keine negative Ladung tragen, zugesetzt werden. D.h. es werden kationische und/oder nichtionische Emulgatoren bzw. kationische und/oder nichtionische Rheologiemodifizierer zur Stabilisierung der Makroemulsion eingesetzt.

Die Emulgatoren werden aus der Gruppe mit einem HLB-Wert im Bereich von 1 bis 20 gewählt.

Bevorzugt umfassen die verwendeten Zubereitungen zwei nichtionische Emulgatoren (b.).

Insbesondere werden nichtionische Emulgatoren gewählt, so dass der Gesamt HLB Wert der Emulgatoren im Bereich von 3 bis 17, bevorzugt 4 bis 16, insbesondere 9 bis 16, insbesondere 10 bis 15, insbesondere im Bereich von 10 bis 14 liegt.

In einer Makroemulsion liegt der Gesamt HLB-Wert der Emulgatoren bevorzugt im Bereich von 4 bis 12, idealerweise im Bereich 7 bis 10.

Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

Es wird dabei unterschieden zwischen nicht-ionischen, anionischen und kationischen Emulgatoren. Ein Kennzeichen für die Hydrophilie eines gegebenen Emulgators ist dessen HLB-Wert, der sich nach folgender Formel ergibt: $HLB = 20 \times (1 - M_{lipophil}/M)$, wobei $M_{lipophil}$ für die Molmasse des lipophilen Anteils im Emulgator und $M$ für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler, die hier auch als Emulgatoren zu wählen sind, bezeichnet.

Beispiele bevorzugter nichtionischer Emulgatoren (b.) sind Glycol Distearate (HLB 1.0), Sorbitan Trioleate (HLB 1.8), Propylene Glycol Isostearate (HLB 2.5), Glycol Stearate (HLB 2.9), Glyceryl Isostearate (HLB 3,5), Sorbitan Sesquioleate (HLB 3.7), Glyceryl Stearate (HLB 3.8), Lecithin (HLB 4), Sorbitan Oleate (HLB 4.3), Sorbitan Monostearate NF (HLB 4.7), Sorbitan Stearate (HLB 4.7), Sorbitan Isostearate (HLB 4.7), Steareth-2 (HLB 4.9), Oleth-2 (HLB 4.9), Glyceryl Laurate (HLB 5.2), Ceteth-2 (HLB 5.3), PEG-30 Dipolyhydroxystearate (HLB 5.5), Glyceryl Stearate SE (HLB 5.8), Sorbitan Stearate (and) Sucrose Cocoate (HLB 6), PEG-4 Dilaurate (HLB 6), PEG-8 Dioleate (HLB 8), Sorbitan Laurate (HLB 8.6), PEG-40 Sorbitan Peroleate (HLB 9), Laureth-4 (HLB 9.7), PEG-7 Glyceryl Cocoate (HLB 10), PEG-20 Almond Glycerides (HLB 10), PEG-25 Hydrogenated Castor Oil (HLB 10.8), Stearamide MEA (HLB 11), Glyceryl Stearate (and) PEG-100 Stearate (HLB 11), Polysorbate 85 (HLB 11), PEG-7 Olivate (HLB 11), Cetearyl Glucoside (HLB 11), PEG-8 Oleate (HLB 11.6), Polyglyceryl-3 Methyglucose Distearate (HLB 12), Oleth-10 (HLB 12.4), Oleth-10 / Polyoxyl 10 Oleyl Ether NF (HLB 12.4), Ceteth-10 (HLB 12.9), PEG-8 Laurate (HLB 13), Ceteareth-12 (HLB 13,5), Cocamide MEA (HLB 13.5), PEG-30 Glyceryl Laurate (HLB 14), Polysorbate 60 NF (HLB 14.9), Polysorbate 60 (HLB 14.9), Polysorbate 80 (HLB 15), PEG-40 Hydrogenated Castor Oil (HLB 15), Isosteareth-20 (HLB 15), PEG-60 Almond Glycerides (HLB 15), Polysorbate 80 NF (HLB 15), PEG-20 Methyl Glucose Sesquistearate (HLB 15), Ceteareth-20 (HLB 15.2), Oleth-20 (HLB 15.3), Steareth-20 (HLB 15.3), Steareth-21 (HLB 15.5),Ceteth-20 (HLB 15.7), Isoceteth-20 (HLB 15.7), Polysorbate 20 (HLB 16.7), Polysorbate 20 NF (HLB 16.7), Laureth-23 (HLB 16.9), PEG-100 Stearate (HLB 18.8), Steareth-100 (HLB 18.8), PEG-80 Sorbitan Laurate (HLB 19.1), PEG-150Laurate(HLB 19.3).

Bevorzugt werden dabei Glyceryl Isostearate, Glyceryl Stearate, Steareth-2, Ceteareth-20, Steareth-21, PEG-40 Hydrogenated Castor Oil, PG-10 Stearate, Isoceteth-20, Isosteareth-20 und Ceteareth-12.

Als Lösungsvermittler bekannt aber als Emulgatoren sind des Weiteren bevorzugt zu wählen PEG-40 Hydrogenated Castor Oil, Polysorbate 80, Laureth-23, PEG-150Laurate und PEG-30 Glyceryl Laurate.

Neben bzw. anstelle nichtionischer Emulgatoren b.) sind auch kationische Emulgatoren c.) geeignet um stabile Formulierungen mit Polyquaternium Polymeren, insbesondere PQ-16 und PQ-6, zu erstellen. Bevorzugte geeignete kationische Emulgatoren c.) sind zu wählen aus der Gruppe Cetrimonium Chloride, Palmitamidopropyltrimonium Chloride, Quaternium-87, Behentrimonium Chloride, Distearoylethyl Dimonium Chloride, Distearyldimonium Chloride, Stearamidopropyl Dimethylamin und/oder Behentrimonium Methosulfat.

Beispiele Rheologiemodifizierer d.) sind natürliche organische Polymere und ihre Derivate, Gummi arabicum, Karaya, Tragant, Johannisbrotkernmehl, Guar, Pektin, Agar agar, Carrageen, Alginate, Xanthan, Stärke und Stärkederivate, Cellulose und Cellulosederivate, wie Microcristalline Cellulose,Methylcellulose, Cellulose Gum, Hydroxyethylcellulose, Hydroxylpropylcellulose, Methylhydroxypropylcellulose, desweiteren anorganische Gelbildner, wie Silikate, z.B. Bentonite, Hectorite, kolloidale Kieselsäure; darüber hinaus synthetische Verdicker z.B. Poylvinylalkohol und ebenso ethoxylierte Verbindungen wie Poloxamer, PEG-150 Distearate, PEG-120 Methyl Glucose Dioleate, PEG-9 Dilaurate und Fettalkohole, wie Stearylalkolhol, Cetylalkohol und Cetearylalkohol.

Werden beispielsweise negativ geladene Stoffe, wie anionische Emulgatoren eingesetzt, so wird keine Stabilisierung, sondern Agglomerationen und Phasenbildungen beobachtet.

Der alleinige Verzicht auf anionische Emulgatoren, Tenside, Solubilisatoren und Rheologiemodifizierer führt dabei nicht automatisch zu den verwendeten stabilen Formulierungen. Vielmehr wurde gefunden, dass es bevorzugt ist eine definierte Polarität des Emulgatorsystems zu wählen (nichtionisch und HLB im bevorzugten Bereich, z.B. 7 bis 16, und/oder kationisch) neben der Abwesenheit von anionischen Ladungen um die verwendeten Zubereitungen, insbesondere mit Polyquaternium-16 und PQ-6, zu stabilisieren.

Es wird daher bevorzugt auf die Anwesenheit organischer Verbindungen mit anionischer Ladung verzichtet und die Emulgatoren im HLB-Wertebereich gewählt.

Erstaunlicherweise konnte eine nochmals verbesserte Stabilität von Polyquaternium Polymeren Polyquaternium-16, Polyquaternium-6 und/oder Polyquaternium-37 und insbesondere PQ-16 oder PQ-6 enthaltene Zubereitungen erreicht werden, wenn der Einsatz von nichtionischen Emulgatoren oder eines Solubilisierers (Lösevermittlers) mit einem HLB-Wert von mindestens 8, bevorzugt mindestens 9, optional in Kombination mit Emulgatoren mit einem HLB-Wert kleiner 8, insbesondere kleiner 9, erfolgt, wobei auch hier der Gesamt HLB Wert aller Emulgatoren und Lösevermittler im bevorzugten Bereich liegt.

Bevorzugt liegt bei einer Kombination von Emulgatoren der lipophilere Emulgator im Verhältnis von 7:1 bis 1:7 zum hydrophileren Emulgator vor, bevorzugt im Verhältnis 6 :1 bis 1:6, bevorzugt im Verhältnis 5:1 bis 1:5, bevorzugt im Verhältnis 4:1 bis 1:4.

Erwünscht und bevorzugt ist ein so resultierender HLB-Wert des gesamten Emulgatorsystems im Bereich von 3 - 15 und bevorzugt von 4 bis 12.

Als Emulgatoren mit einem HLB-Wert kleiner 9 werden beispielsweise Glyceryl Isostearate, Glyceryl Stearate und Steareth-2 bevorzugt.

Als hydrophilere Emulgatoren mit einem HLB-Wert von mindestens 9 werden bevorzugt Ceteareth-20, Steareth-21, PEG-40 Hydrogenated Castor Oil, PG-10 Stearate, Isoceteth-20, Isosteareth-20 und Ceteareth-12.

Bei transparenten Mikroemulsionen und opaken Makroemulsion zeigt sich diese optimale Kombination von Emulgator und Co-Emulgator stabilisierend, optional durch additiven Einsatz eines nichtionischen/kationischen Rheologiemodifizierers. D.h. bei einer transparenten Mikro- oder Makroemulsion sind zwei nichtionische Emulgatoren und optional ein Rheologiemodifizierer zuzusetzen.

Der Zusatz an PQ-16 in transluzenten PIT-Formulierungen konnte ebenfalls gelingen, da diese, wie bei der Mikro- und Makroemulsion, durch Zusatz an nichtionischen Emulgatoren stabilisiert werden können.

Bei wässrig-alkoholischen Formulierungen zeigt sich für die Stabilisierung der Zusatz eines Lösungsvermittlers erfolgsversprechend. Insbesondere konnte dabei der Anteil an C1-C6-Alkoholen, insbesondere ein Ethanolgehalt, reduziert werden. Ein Ethanolgehalt von 30% bis zu 45% ist hierbei ausreichend, obwohl im Stand der Technik nur Zubereitungen mit einem Alkoholgehalt von 50% und mehr offenbart werden.

Optional werden hier mindestens ein Emulgator b.) mit einem HLB - Wert von größer 9 und/oder Rheologiemodifizierer d.) zur Stabilisierung oder zur Veränderung der Viskosität eingesetzt.

Die verwendeten Zubereitungen zeigen ein homogenes Erscheinungsbild, was dadurch gekennzeichnet ist, dass sich keine Ausfällungen, Phasenbildungen oder Kristallbildung zeigen.

Die Zubereitungen können sowohl transparent, beispielsweise als Mikroemulsion, alkoholische Lösung, als auch transluzent, z.B. als PIT-Emulsion oder opak, z.B. als Makroemulsion, formuliert werden.

Darüber hinaus konnte überraschend festgestellt werden, dass auch die Auswahl der Ölkomponente einen entscheidenden Einfluss auf die Lagerstabilität, insbesondere von Makroemulsionen, besonders bei thermischer Belastung, hat. Besonders bewährt haben sich erfindungsgemäß Triglyceride.

Bevorzugte Triglceride sind zu wählen aus der Gruppe Acetic/Linoleic/Palmitic Triglyceride, C8-12 Acid Triglyceride, C12-18 Acid Triglyceride, C18-36 Acid Triglyceride, Capric/Lauric/Myristic/Oleic Triglyceride, Caprylic/Capric/Lauric Triglyceride, Caprylic/Capric/Linoleic Triglyceride, Caprylic/Capric/Myristic/Stearic Triglyceride, Caprylic/Capric/Palmitic/Stearic Triglyceride, Caprylic/Capric/Stearic Triglyceride, Caprylic/Capric/Succinic Triglyceride, Caprylic/Capric Triglyceride, Caprylic/Capric Triglyceride PEG-4 Esters, C10-40 Isoalkyl Acid Triglyceride, Cod Liver/Mink/Tallow Triglyceride, C10-18 Triglycerides, C10-18 Triglycerides Polyglyceryl-3 Esters Phosphates, Docosahexenoic/Docosapentenoic/Oleic/Palmitic Triglyceride, Hydrogenated C12-18 Triglycerides, Jojoba Oil/Caprylic/Capric Triglyceride Esters, Lauric/Palmitic/Oleic Triglyceride, Mustelic/Palmitic Triglyceride, Oleic/Linoleic Triglyceride, Oleic/Palmitic/Lauric/Myristic/Linoleic Triglyceride, Palmitic/Stearic Triglyceride und Ricinoleic/Caproic/Caprylic/Capric Triglyceride.

Bevorzugt zu wählen sind mittelkettige Triglyceride, bevorzugt das Lipid Caprylic/Capric Triglyceride.

So wurde anhand des Austauschs der Ölkomponente PPG-15 Stearylether gegen Caprylic/Capric Triglyceride eine weiter stabilisierte Zubereitung erhalten. Triglyceride sind demnach für die Stabilität, gerade unter thermischer Belastung, besonders förderlich.

Ebenfalls überraschend zeigten sich die verwendeten Zubereitungen umfassend ein oder mehrere Parfumbestandteile auch hinsichtlich der Parfumstabilität verbessert. D.h. es ist ein längerer Parfumeffekt bemerkbar gegenüber Zubereitungen ohne Zusatzstoffe b. (nichtionische Emulgatoren), c. (kationische Emulgatoren) oder d. (Rheologiemodifizierer).

Eine Zersetzung des Parfums findet nicht statt.

Als besonders bevorzugte, weil weiter stabilisierend und verstärkt desodorierend, haben sich die Parfumkomponenten Terpin-4-ol (CAS 562-74-3), Linalool (78-70-6), Citral, Geraniol, Neroll, Perillaldehyde, $\alpha$-Terpineol, Thymol, Eugenol, Citronellal, Terpinyl Acetate, Citronellol" $\beta$-Pinene, Benzaldehyde, 4-Methylbenzaldehyde, Heliotropine, Vanillin, 3-Hydroxy-4-methoxybenzaldehyde (Isovanillin), Geranium oil, Peppermint oil, Rose oil, Cinnamon leaf oil, Fucus oil, Clove bud oil, Clove leaf oil, Palmarosa oil, Citrus oil, Tepene fraction of citrus oil, Orange oil, Terpene fraction of orange oil, Cuminic alcohol, Perilla alcohol,Cinnamic alcohol,Limonene, Dihydroterpineol, Citral,Eucalyptol und Eugenol erwiesen.

Die Zubereitungen umfassen bevorzugt keine antitranspirant-wirksamen Substanzen, insbesondere keine Aluminiumsalze, wie Aluminiumchlorohydrate.

Die Molmasse von PQ-16 Polymeren liegt bevorzugt zwischen 10.000 - 1.000.000 [Da].

Bei der Methode der Molmassenbestimmung handelt es sich um eine GPC mit wässrigem Eluentensystem, kalibriert mit Dextran/Pullulan-Standards.

Methodeninformation

Kalibrationsart: Conventional
Int. Standard - K: 50,00 ml
Int. Standard - M: 0,00 ml
Probenkonzentration: 3,00 g/l
Injektvolumen: 50 $\mu$l
Eluent: 0.1 M NaCl / 0.1% TFA
Flussrate: 1,000 ml/min
Säulen: PSS Novema 10$\mu$m 30,300,1000
Temperatur: 25,0 °C
GPC-Anlage: PG13

Nach folgende Vergleichsuntersuchchungen zeigen die bemerkenswerten Vorteile der Zubereitungen.

1) Stabilisierung von Mikroemulsionsgelen

In Standard-Mikroemulsionen, wie sie beispielsweise in WO 1998015254 A1 beschrieben sind, wird Polyquaternium-16 eingearbeitet und so die Zubereitung durch ein Verhältnis von Emulgator zu Co-Emulgator stabilisiert. Erstaunlicherweise wurde gegenüber den bekannten Zubereitungen, die C1-C6-Alkoholen umfassen, ohne diese C1-C6 Alkohole dennoch eine antimikrobielle Wirksamkeit erreicht, wie in den angefügten Untersuchungen dargelegt ist.

| INCI: O/W Emulsion | 1 | 2 | 3 | 4 | 5 | 6 | 7 |
|---|---|---|---|---|---|---|---|
| Persea Gratissima Oil | 0,5 | 0,1 | 0 | 0,1 | 0 | 0,1 | 0,1 |
| Pionier 2076 (Paraffinum Liquidum; Hansen & Rosenthal) | 4 | 3 | 0 | 3 | 2,8 | 0 | 3 |

(fortgesetzt)

| INCI: O/W Emulsion | 1 | 2 | 3 | 4 | 5 | 6 | 7 |
|---|---|---|---|---|---|---|---|
| Tegin ISO (Glyceryl Isostearate; Evonik Industries) | 2,0 | 2,2 | 2,4 | 2,4 | 1,5 | 2,1 | 2,2 |
| Tego Alkanol IC 20 (Isoceteth-20; Evonik Industries) | 4,2 | 5 | 4,8 | 4,7 | 3,65 | 5 | 5 |
| Cetiol OE (Dicaprylyl Ether, BASF Personal Care and Nutrition) | 0 | 0 | 3 | 0 | 0 | 2 | 0 |
| Glycerin | 0 | 2 | 0 | 0 | 0 | 2 | 2 |
| Trisodium EDTA | 0,5 | 0,2 | 0,5 | 0,5 | 0,2 | 0,2 | 0,2 |
| Octopirox (Piroctone Olamine; Clariant) | 0,05 | 0,05 | 0,05 | 0,05 | 0,05 | 0,05 | 0,05 |
| Parfum | q.s. | q.s | q.s | q.s | q.s | q.s | q.s |
| Butylene Glycol | 4 | 3 | 3 | 3 | 3 | 3 | 3 |
| Kessco PEG 6000 DS HV (PEG-150 Distearate; Italmatch Chemical) | 0,5 | 0,75 | 0,7 | 0,7 | 1,2 | 0,7 | 0,85 |
| Luviquat Excellence (Polyquaternium-16; BASF) | 1,5 | 2,5 | 0 | 0 | 0 | 0 | 2,5 |
| Luviquat FC 550 (Polyquaternium-16; BASF) | 0 | 0 | 1,25 | 4 | 0 | 0 | 0 |
| Luviquat FC 370 (Polyquaternium-16; BASF) | 0 | 0 | 0 | 0 | 3,5 | 0 | 0 |
| Luviquat Style (Polyquaternium-16; BASF) | 0 | 0 | 0 | 0 | 0 | 3,5 | 0 |
| Aqua | ad 100 | | | | | | |
| Resultat: Homogene Zubereitung | | | | | | | |

Homogen bedeutet dabei keinerlei Ausfällungen, Agglomerationen, Phasenbildungen.

Die Emulgatorsysteme weisen einen HLB - Wert im Bereich von 10 bis 14 auf.

2) Stabilisierung von Makroemulsionen

| INCI O/W Emulsion | 8 | 9 | 10 | 11 |
|---|---|---|---|---|
| Persea Gratissima Oil | 0,1 | 0,1 | 0,25 | 0,2 |
| Cetiol E ( PPG-15 Stearyl Ether; BASF Personal Care and Nutrition) | 4 | 4 | 3 | 4 |
| Tego Alkanol S 21 (Steareth-21; Evonik Industries) | 2,5 | 2,3 | 2,0 | 2,6 |
| Tego Alkanol S2 (Steareth-2; Evonik Industries) | 1,5 | 1,7 | 2,0 | 1,7 |
| Parfum | q.s. | q.s. | q.s. | q.s. |
| Trisodium EDTA | 0,3 | 0,3 | 0,3 | 0,3 |
| Piroctone Olamine (Octopirox; Clariant) | 0,05 | 0,05 | 0,05 | 0,05 |
| Natrosol 250 HHX pharm (Hydroxyethylcellulose; Ashland Aqualon Functional Ingredients) | 0,35 | 0,45 | 0,45 | 0,35 |
| Luviquat Excellence (Polyquaternium-16; BASF) | 1,5 | 0 | 0 | 0 |

(fortgesetzt)

| INCI O/W Emulsion | 8 | 9 | 10 | 11 |
|---|---|---|---|---|
| Luviquat FC 550 (Polyquaternium-16; BASF) | 0 | 2 | 0 | 0 |
| Luviquat FC 370 (Polyquaternium-16; BASF) | 0 | 0 | 1 | 0 |
| Luviquat Style (Polyquaternium-16; BASF) | 0 | 0 | 0 | 1,5 |
| Aqua | ad 100 | | | |
| Resultat: Homogene Zubereitung | | | | |

| INCI O/W Emulsion | 12 | 13 | 14 | 15 | 16 |
|---|---|---|---|---|---|
| Persea Gratissima Oil | 0,1 | 0,1 | 0,1 | 0,2 | 0,3 |
| Myritol 312 (Caprylic/Capric Triglyceride; BASF Personal Care and Nutrition) | 4 | 4 | 3 | 4 | 3 |
| Tego Alkanol S 21 (Steareth-21; Evonik Industries) | 0,5 | 1 | 2 | 2 | 4 |
| Tego Alkanol S2 (Steareth-2; Evonik Industries) | 5 | 4 | 3,5 | 3 | 2 |
| Parfum | q.s. | q.s. | q.s. | q.s. | q.s. |
| Trisodium EDTA | 0,3 | 0,3 | 0,3 | 0,3 | 0,3 |
| 100981 Benzyl alcohol EMPROVE exp Ph Eur,BP,JP,NF ( Benzyl Alcohol; Merck) | 0,3 | 0,2 | 0,3 | 0,5 | 0,25 |
| Phenoxyethanol R + (Phenoxyethanol; Univar) | 0,7 | 0,5 | 0,5 | 0,2 | 0,5 |
| Glycerol EP vegetable (Glycerin, Spiga Nord) | 5 | 8 | 5 | 4 | 5 |
| Luviquat Excellence (Polyquaternium-16; BASF) | 1,5 | 0 | 2,5 | 0 | 1,5 |
| Luviquat FC 550 (Polyquaternium-16; BASF) | 0 | 2 | 0 | 0 | 0 |
| Luviquat FC 370 (Polyquaternium-16; BASF) | 0 | 0 | 0 | 1 | 0 |
| Luviquat Style (Polyquaternium-16; BASF) | 0 | 0 | 0 | 1,5 | 0 |
| Aqua | ad 100 | | | | |
| Resultat: Homogene Zubereitung | | | | | |
| HLB | 5,85 | 7,02 | 8,7 | 9,14 | 11,85 |

Die dargestellten Makroemulsionen sind mit einem HLB des Emulgatorsystems von 4 bis 12 stabil, d.h. keine Ausfällungen, kein olfaktorischen Veränderungen.

3) Stabilisierung in alkoholischer Lösung

Für die Stabilisierung von Zubereitungen umfassend Polyquaternium Verbindungen ist nicht zwingend eine Emulsion erforderlich. Auch durch Einsatz von beispielsweise PQ-16 in einer optional mit einem Rheologiemodifizierer verdickten alkoholischen Lösung und mindestens einem Emulgator, bevorzugt nichtionisch mit einem HLB von größer 9, wird eine stabile Zubereitung erhalten.

Überraschend wird dadurch eine Stabilisierung schon mit einem Alkoholgehalt unter 50% erreicht, bevorzugt 45% bzw. 30%, wohingegen im Stand der Technik (US 20070184016 A1, WO2007095008 A2) der Alkoholgehalt mindestens 50% beträgt.

| INCI: Alkoholische Lösung | 17 Roll-on | 18 Zerstäuber | 19 Roll-on | 20 Zerstäuber | 21 Roll-on | 22 Zerstäuber | 23 Roll-on | 23b Zerstäuber |
|---|---|---|---|---|---|---|---|---|
| Citric Acid | 0,05 | 0,1 | 0,05 | 0,05 | 0,05 | 0,1 | 0,05 | 0,1 |
| Natrosol 250 HHX pharm (Hydroxyethylcellulose; Ashland Aqualon Functional Ingredients) | 0,2 | 0 | 0,3 | 0 | 0,35 | 0 | 0,2 | 0 |
| Eutanol G (Octyldodecanol; BASF Personal Care and Nutrition) | 0,1 | 0,1 | 0,1 | 0,1 | 0,1 | 0,1 | 0,1 | 0,1 |
| Ethanol | 30 | 45 | 30 | 45 | 30 | 45 | 30 | 45 |
| Parfum | q.s. | q.s. | q.s. | q.s. | q.s. | q.s. | q.s. | q.s. |
| Polyglykol 400 (PEG-8; Clariant) | 1,0 | 0 | 1,0 | 0 | 2,0 | 0 | 1,0 | 0 |
| Cremophor RH 410 ( PEG-40 Hydrogenated Castor Oil; BASF) | 1,5 | 1 | 1,5 | 1 | 2,0 | 1 | 1,5 | 1 |
| Luviquat Excellence (Polyquaternium-16; BASF) | 0 | 0 | 0 | 2,5 | 2,5 | 0 | 0 | 0 |
| Luviquat FC 550 (Polyquaternium-16; BASF) | 0 | 2 | 3 | 0 | 0 | 0 | 0 | 0 |
| Luviquat FC 370 (Polyquaternium-16; BASF) | 4 | 0 | 0 | 0 | 0 | 3 | 0 | 0 |
| Luviquat Style (Polyquaternium-16; BASF) | 0 | 0 | 0 | 0 | 0 | 0 | 2 | 3 |
| Aqua | Ad 100 | | | | | | | |
| Resultat: Homogene Zubereitung | | | | | | | | |

In anderen Zubereitungen ist ein homogenes Erscheinungsbild bei Anwesenheit von Polyquaternium-16 nicht möglich, wie die nachfolgenden Rezepturbeispiele 25 bis 30 zeigen.

Liegt wie hier kein optimales Verhältnis von Emulgator zu Co-Emulgator vor bzw. liegt der HLB des Emulgators bzw. des Solubilisierers bzw. der Kombination der Emulgatoren nicht im optimalen Bereich, sind Agglomerationen, Phasenbildungen bzw. eine Destabilisierung der Formulierung zu beobachten.

| INCI: O/W Emulsion | 25 | 26 | 27 | 28 |
|---|---|---|---|---|
| Tegin VS (Glyceryl Stearate SE; Evonik Industries) | 2,0 | 1,8 | 2,0 | 2,1 |
| Lanette E granules (Sodium Cetearyl Sulfate; BASF Personal Care and Nutrition) | 0,5 | 0,7 | 0,5 | 0,4 |
| Parfum | q.s. | q.s. | q.s. | q.s. |
| Glycerin | 7 | 7 | 7 | 7 |
| Sodium Hydroxide | 0,5 | 0,5 | 0,5 | 0,5 |
| Phenoxyethanol | q.s. | q.s. | q.s. | q.s. |
| Methylisothiazolinone | q.s. | q.s. | q.s. | q.s. |
| Carbopol 981 (Carbomer; Lubrizol Advanced Materials) | 0,25 | 0,3 | 0,25 | 0,25 |

(fortgesetzt)

| INCI: O/W Emulsion | 25 | 26 | 27 | 28 |
|---|---|---|---|---|
| Alcohol Denat. | 6 | 5 | 8 | 7 |
| Luviquat Excellence (Polyquaternium-16; BASF) | 1,5 | 0 | 0 | 0 |
| Luviquat FC 550 (Polyquaternium-16; BASF) | 0 | 2 | 0 | 0 |
| Luviquat FC 370 (Polyquaternium-16; BASF) | 0 | 0 | 1 | 0 |
| Luviquat Style (Polyquaternium-16; BASF) | 0 | 0 | 0 | 1,5 |
| Aqua | ad 100 | | | |
| Resultat | Agglomeration | | | |

Agglomerationen, Phasenbildungen bzw. eine Destabilisierung der Formulierung sind zu beobachten. Bei folgenden opake Formulierungen treten bei thermischer Belastung Instabilitäten auf:

| INCI O/W Emulsion | 29 | 30 |
|---|---|---|
| Persea Gratissima Oil | 0,1 | 0,1 |
| Cetiol E ( PPG-15 Stearyl Ether; BASF Personal Care and Nutrition) | 4 | 0 |
| Benzoate DE C12/C15 (C12-15 Alkyl Benzoate; Stearinerie Dubois) | 0 | 3 |
| Tego Alkanol S 21 (Steareth-21; Evonik Industries) | 1,5 | 1,2 |
| Tego Alkanol S2 (Steareth-2; Evonik Industries) | 2,5 | 2,8 |
| Parfum | q.s. | q.s. |
| Trisodium EDTA | 1,5 | 1,5 |
| 100981 Benzyl alcohol EMPROVE exp Ph Eur,BP,JP,NF ( Benzyl Alcohol; Merck) | 0,3 | 0,2 |
| Phenoxyethanol R + (Phenoxyethanol; Univar) | 0,7 | 0,5 |
| Luviquat Excellence (Polyquaternium-16; BASF) | 3 | 2 |
| Aqua | ad 100 | |
| Resultat: Phasentrennung | | |

Überraschenderweise zeigen die Polyquaternium Polymere ausgewählt aus der Gruppe Polyquaternium-16, Polyquaternium-6 und/oder Polyquaternium-37, insbesondere Polyquaternium-16 Polymere und Polyquaternium-6 Polymere, gegenüber den Bakterien Staphylococcus epidermidis und Corynebacterium jeikeium eine antimikrobielle Wirksamkeit und damit eine Deo-wirkung.

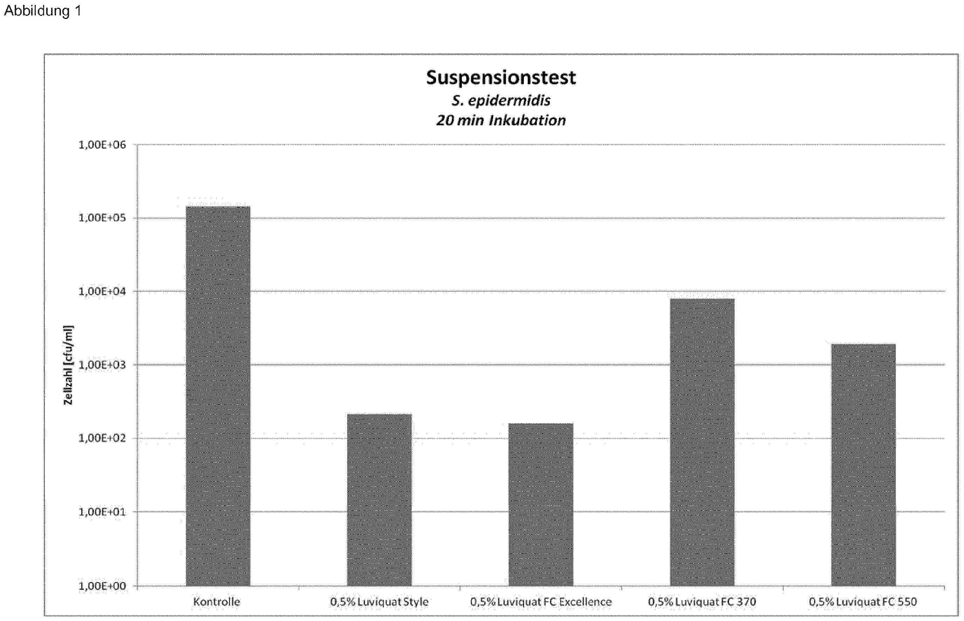

Im einem Suspensionstest (s. Abbildung 1) wurden verschiedene PQ-16 Varianten untersucht. Die erfindungsgemäßen PQ-16 Verbindungen zeigen eine antimikrobielle Wirksamkeit und Deo-wirkung.

Andere antimikrobielle Stoffe, wie beispielsweise Triclosan, der durchaus als antimikrobieller Stoff bekannt ist, zeigt gegen diese Deo-relevanten Bakterien Staphylococcus epidermidis und Corynebacterium jeikeium keine bzw. nicht ausreichende Wirksamkeit, wie auch in J. Soc. Cosmet. Chem., 38, 223-231 (July/August 1987) - Efficacy of the antimicrobial agent triclosan in topical deodorant products - gezeigt wurde.

Es zeigt sich, dass Triclosan keine antimikrobielle Wirksamkeit in der Achsel gegenüber dem ethanolischem Aerosolspray zeigt, zwar eine geringe Wirksamkeit nach 12h im Sniff-Test, aber keine Wirksamkeit nach 24h im Sniff-Test.

Verwendete Zubereitungen zeigen sowohl nach Stunden aus beispielsweise einem ethanolischem Pumpspray (45% Ethanol) eine deutliche antimikrobielle Wirksamkeit in der Achsel und eine 24h Wirksamkeit gegenüber dem Vehikel im Sniff-Test.

Es ist daher nicht allein aus der Kenntnis einer allgemeinen antimikrobiellen Wirksamkeit gleichzeitig auf eine gute bzw. überhaupt vorhandene desodorierende Wirksamkeit zu schließen.

Für eine Deowirksamkeit müssen die entscheidenden Bakterien, wie Bakterien Staphylococcus epidermidis, signifikant reduziert werden.

Dazu wurden Suspensionstests wie folgt durchgeführt:

Das Bakterium *S. epidermidis (ATCC 12228)* wurde aus einem nach EN 12353 angelegten Cryoröhrchen auf einer entsprechenden Agarplatte (TSA-Agar) ausgestrichen und bei 37°C 24 Stunden bebrütet. Von den gewachsenen Bakterien wurde eine zweite Passage angelegt, die im Suspensionstest eingesetzt wurde.

Eine Impföse der Bakterien wurde zu 10mL LB-Medium (n.G. Bertani) und 5g Glasperlen mit 4mm Durchmesser gegeben und 3 Minuten gevortext. Anschließend wurde diese Bakteriensuspension auf eine OD (optischen Dichte) von 0,08 eingestellt. Die eingestellte Bakteriensuspension wurde dann 1:20 mit LB-Medium verdünnt, um eine Keimzahl von ungefähr $5*10^5$ zu gewährleisten. Diese Bakteriensuspension wurde anschließend für den Test eingesetzt.

Der Suspensionstest wurde per Hand durchgeführt. Hierzu wurden 900μL der zu testenden Wirkstofflösungen bzw. im Falle der Kontrollen 900 μl Phophatpuffer in Eppendorfgefäßen vorgelegt. Anschließend wurden 100 μl der Bakteriensuspension hinzugegeben und gevortext. Zum Zeitpunkt $t_0$ wurde eine Probe aus den Kontrollen entnommen. Die Eppendorfgefäße wurden nun im Thermoschüttler bei 37 °C inkubiert. Nach 20, 60 und 180 Minuten bei 37°C und 1200rpm wurden aus den Eppendorfgefäßen im Thermoschüttler jeweils 100μL entnommen und zu 900μL LB-Medium pipettiert ($10^{-1}$-Verdünnung). Diese Verdünnungen wurden mittels Spiralplatter auf Agarplatten ausplattiert. Eine Ausnahme stellt hier die Kontrolle dar, die nochmal 1:100 verdünnt wurde ($10^{-3}$-Verdünnug). Bei den Kontrollen wurden beide Verdünnungen ausplattiert, einschließlich des Zeitpunkts T=0 Minuten. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

$$\frac{CFU(Kontrolle)}{CFU(Testsubstanz)} = Reduktionsfaktor$$

| Wirkstofflösung | Reduktionsfaktor 20 Min |
|---|---|
| 0,5% Luviquat FC Excellence | 919 |
| 0,5% Luviquat FC 550 | 77 |
| 0,5% Luviquat FC 370 | 19 |
| 0,5% Luviquat Style | 689 |

Die angegeben Prozentgehalte beziehen sich auf den Gewichtsanteil an Aktivsubstanz, also ohne Lösemittel Wasser, im Phosphatpuffer.

Die Rohstoffe wurden in Phosphatpuffer gelöst.

Phosphatpuffer umfasst:

A) Kaliumdihydrogenphosphat 9,078 g/L

B) Dinatriumhydrgenphosphat *2 $H_2O$ 11,876 g/L

122 mL A zu 363 mL B = Gebrauchslösung

Es zeigte sich, dass die erfindungsgemäßen Polyquaternium Polymere zu einer entscheidenden Bakterienreduktion führen und somit eine Deowirksamkeit zeigen.

Als desodorierend wirksam wird ein Reduktionsfaktor von mindestens 10 angesehen.

Insbesondere Polyquaternium-16 Polymere mit einem VP/QVI Verhältnis im Bereich von 5 zu 95 (Luviquat Excellence) zeigen eine besonders gute desodorierende Wirkung, die so nicht zu erwarten war (Reduktionsfaktor 919).

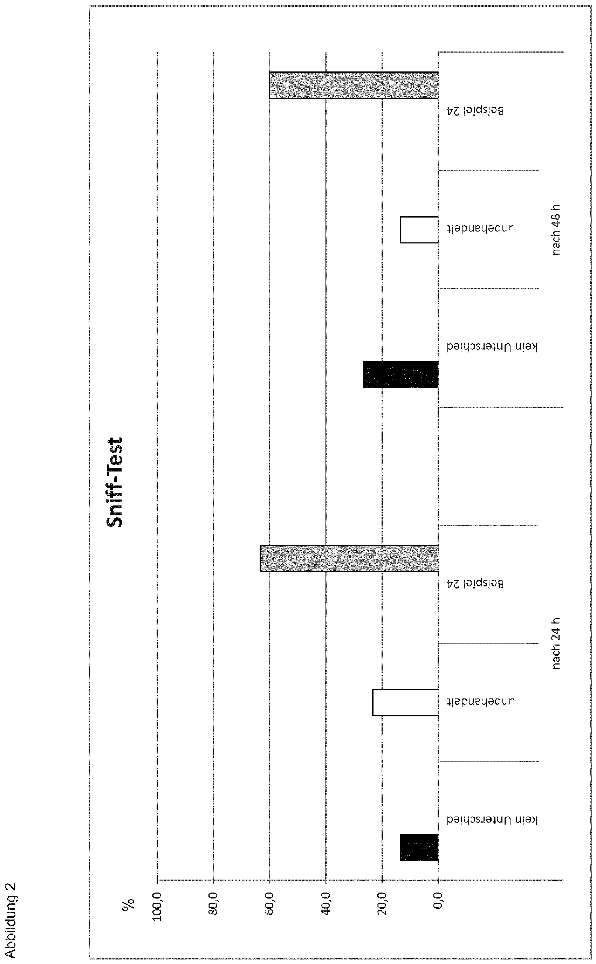

In einem Sniff-Test (s. Abbildung 2) zeigte sich ebenso eine signifikante Geruchsreduktion aus einem unparfümierten Mikroemulsionsgel (Beispiel 24).

1% Polyquaternium-16 erzielt nach einer Einmalapplikation eine deutliche Geruchsreduktion gegenüber der unbehandelten Achsel.

Der Sniff Test (SNK) dient der Bestimmung einer i.d.R. 48-stündigen desodorierenden Wirksamkeit von kosmetischen Formulierungen nach einmaliger Anwendung. Die Anwendung des Testproduktes und die Messung der Wirksamkeit erfolgen in dem verbraucherrelevanten Areal, der Achsel. Zielgröße ist die Geruchsintensität des Achselschweißes.

Messung

Die Bestimmung der deodorierenden Wirksamkeit wird über die Verwendung valider und anerkannter Messmethoden und Beurteilungsverfahren realisiert.

Das Studiendesign ist so ausgelegt, dass deodorierende Wirksamkeiten wie folgt nachgewiesen werden können:

- zumindest einfach verblindet und randomisiert in Bezug auf die Prüfmuster und offen für die unbehandelte Kontrolle
- Vergleich zur unbehandelten Kontrolle -> Wirknachweis für Wirkstoff & Formulierung,

Die Messung erfolgt durch eine subjektive, olfaktorische Beurteilung der Intensität des Achselschweißes durch ein Sniffer-Panel (weibliche und männliche Probanden). Die Beurteilung der Geruchsintensität des Achselschweißes erfolgt i.d.R. für eine vergleichende Beurteilung der Geruchsproben nach den absoluten Skalenwerten von 0 (kein Schweißgeruch) bis 5 (sehr starker Schweißgeruch).

Geprüft wird ein codiertes Prüfmuster (Codierung gemäß WI 7 MAT 100) im kontralateralen Vergleich gegen die unbehandelte Kontrolle.

Nach 24h konnte bei 63,3% der Probanden in der mit der erfindungsgemäßen Zubereitung behandelten Achsel eine signifikante Reduktion des Schweißgeruchs durch das Sniffer-Panel wahrgenommen werden. Bei 13,3% der Probanden konnte kein Unterschied festgestellt werden. Bei 23,3% der Probanden wurde die unbehandelte Achsel besser bewertet. Nach 48h konnte bei 60,0% der Probanden in der mit der erfindungsgemäßen Zubereitung behandelten Achsel eine signifikante Reduktion des Schweißgeruchs durch das Sniffer-Panel wahrgenommen werden. Bei 26,7% der Probanden konnte kein Unterschied festgestellt werden. Bei 13,3% der Probanden wurde die unbehandelte Achsel besser bewertet (s. Abbildung 2).

1% Polyquaternium-16 erzielt nach einer Einmalapplikation daher eine deutliche Geruchsreduktion gegenüber der unbehandelten Achsel.

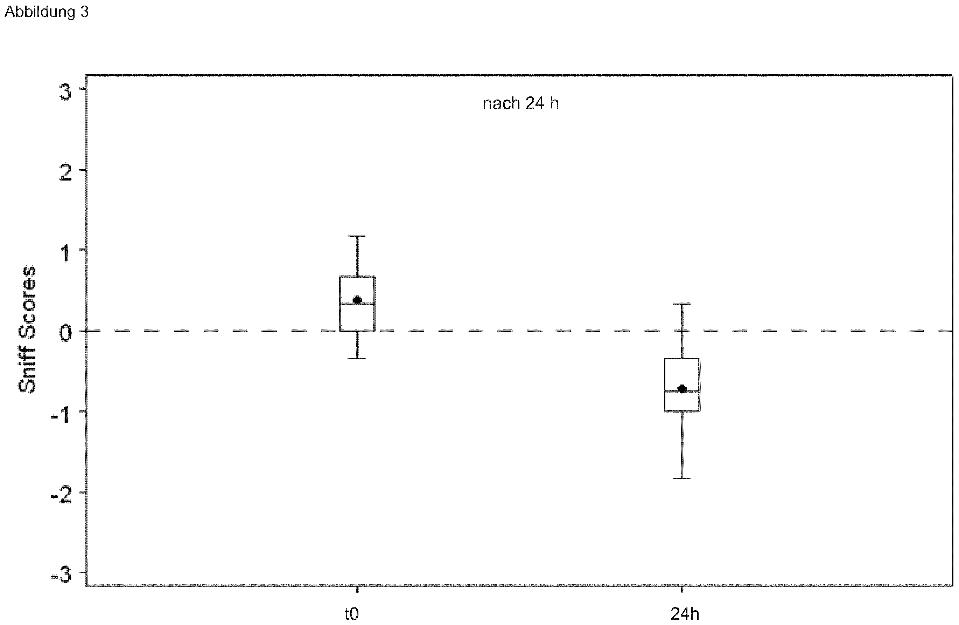

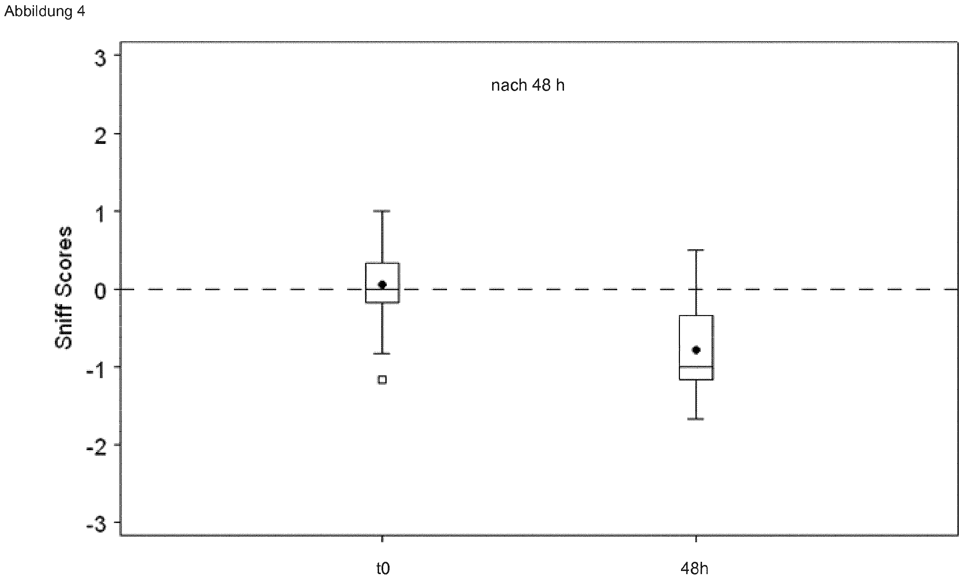

In einem gleich durchgeführten Sniff-Test wurde auch für Polyquaternium-6 eine Deowirkung ermittelt. Mit PQ-6 konnte aus einer unparfümierten Roll-on Formulierung eine 48-h Deowirksamkeit im Sniff-Test nachgewiesen werden (Abbildung 3 und 4).

Es wurde dazu eine Mikroemulsion (Beispiel 58) mit 3,0% MIRAPOL 100 (enthaltend 40% PQ-6 in Wasser) getestet.

| INCI: Mikroemulsion | 58 |
|---|---|
| Pionier 2076 (Paraffinum Liquidum; Hansen & Rosenthal) | 3 |
| Tegin ISO (Glyceryl Isostearate; Evonik Industries) | 2,2 |
| Tego Alkanol IC 20 (Isoceteth-20; Evonik Industries) | 5 |
| Glycerin | 3 |
| Parfum | q.s. |
| Butylene Glycol | 3 |
| Kessco PEG 6000 DS HV (PEG-150 Distearate; Italmatch Chemical) | 0,85 |
| Mirapol 100 (Polyquaternium-6; Solvay) | 3,0 |
| Aqua | ad 100 |
| Resultat: Homogene Zubereitung | |

Getestet wurde analog zum obigen SNIFF-Test.

Nimmt der Schweißgeruch im Laufe des Tests ab, sinkt der SNIFF-Score und die Differenz wird negativ. Beispielsweise Score 3 (nachher) - 5 (vorher)= -2.

Das bedeutet: ist das Vorzeichen der Differenz negativ, wurde der Geruch verbessert. Je "größer" die Differenz bzw. der Zahlenwert, desto stärker die Verbesserung. Erfahrungswerte zeigen, dass einer Differenz von mehr als -0,2 eine signifikante Geruchsverbesserung dokumentieren.

Bei einer Differenz mit positiven Werten hat der Geruch im Laufe des Tests zugenommen.

In dem Sniff-Test (s. Abbildung 3 und 4) zeigte sich ebenso eine signifikante Geruchsreduktion aus einem unparfümierten Mikroemulsionsgel (Beispiel 58). 1,2% Polyquaternium-6 erzielt nach einer Einmalapplikation eine deutliche Geruchsreduktion gegenüber der unbehandelten Achsel.

Nach 24h ist eine signifikante Reduktion des Schweißgeruchs in der mit der verwendeten Zubereitung behandelten Achsel im Vergleich zur unbehandelten Achsel wahrnehmbar. Betrug die Differenz der SNIFF-Scores der behandelten Achsel zur unbehandelten Achsel zu Beginn des Testzeitraumes noch 0,37 wird nach 24h eine Differenz von -0,73 erreicht (Abbildung 3).

Eine Schweißreduktion ist bei einer Differenz von > -0,2 wahrnehmbar (mathematisch kleiner).

Auch nach 48h ist eine signifikante Reduktion des Schweißgeruchs in der mit der verwendeten Zubereitung behandelten Achsel im Vergleich zur unbehandelten Achsel wahrnehmbar. Betrug die Differenz der SNIFF-Scores der behandelten Achsel zur unbehandelten Achsel zu Beginn des Testzeitraumes noch 0,06 wird nach 24h eine Differenz von sogar -0,78 erreicht (Abbildung 4).

Die Formel hat den Test demnach zu beiden Zeitpunkten überraschend aussagekräftig bestanden.

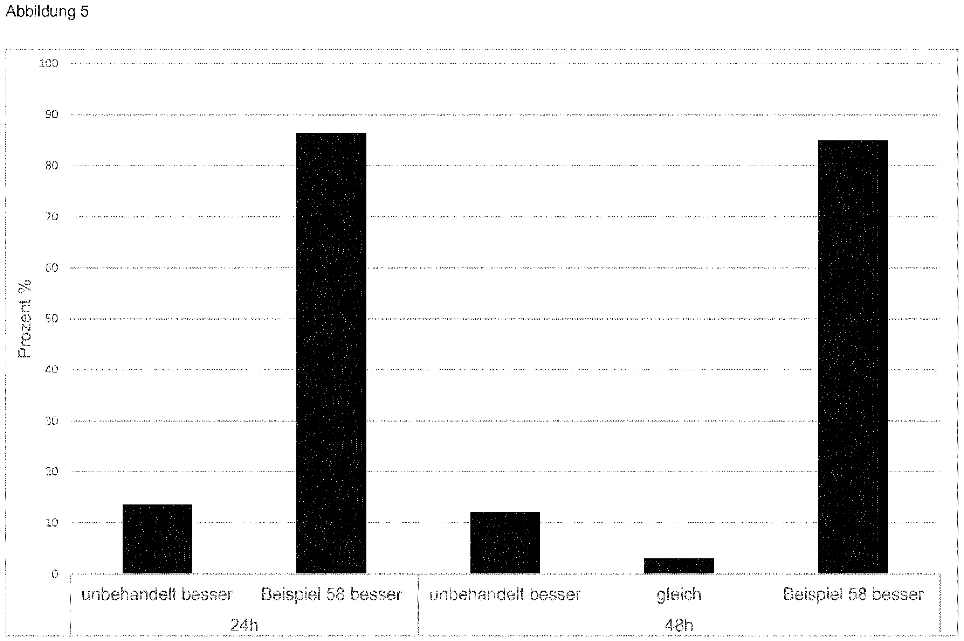

In der Panelauswertung des Sniff-Test ergab sich, dass nach 24h bei 86,4% der Probanden in der mit der verwendeten Zubereitung 58 behandelten Achsel eine signifikante Reduktion des Schweißgeruchs durch das Sniffer-Panel wahrgenommen werden. Bei 13,6% der Probanden wurde die unbehandelte Achsel besser bewertet. Nach 48 Stunden ergab sich bei 84,9% der Probanden eine signifikante Geruchsreduktion, 3% konnten keinen Unterschied feststellen und bei 12,1% der Probanden wurde die unbehandelte Achsel besser bewertet (s. Abbildung 5).

Gleiche Tests wurden auch zur Ermittlung des Deowirksamkeit auf Textilien durchgeführt.

In einem Test wurde die Deowirksamkeit gegen das Bakterium S. epidermidis (ATCC 12228) ermittelt. Die Untersuchungen erfolgte entsprechend der Bestimmung der antibakteriellen Wirkung antibakteriell behandelter Erzeugnisse (ISO 20743:2007).

Die wie zuvor dargestellte Bakteriensuspension wurde anschließend für den Test eingesetzt.

Hierzu wurden 30µL der zu testenden Wirkstofflösungen bzw. im Falle der Kontrollen 30 µl Wasser auf Baumwoll- und Polyester Stanzen pipettiert und 1 h trocknen gelassen. Anschließend wurde 50 µl der Bakteriensuspension hinzugegeben. Die Textilproben wurden dann im Liconic Inkubator bei 37°C und 90% Luftfeuchtigkeit inkubiert. Nach 60 Minuten wurden die Textilproben in Eppendorfgefäße überführt, die 1 ml Neutralisationsmedium enthalten. Nach 1 Minute im Thermoschüttler und unter Intervallschüttlung (5 Sekunden/ 3 Sekunden) wurden die Textilproben entfernt und die Lösung mittels Spiralplatter auf Agarplatten ausplattiert. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

$$\frac{CFU\,(Kontrolle)}{CFU\,(Testsubstanz)} = Reduktionsfaktor$$

| Wirkstofflösung (50 µg/cm$^2$ Textil) | Reduktionsfaktor 20 Min |
|---|---|
| Alexidine (1,1'-(1,6-Hexanediyl)bis{2-[N'-(2-ethylhexyl)carbamimidoyl]guanidine}) | 6 |
| Polyquaternium-37 (AMP019) | 12 |
| Polyquaternium-16 (Luviquat Excellence) | 1335 |
| Polyaminopropyl Biguanide (Cosmocil CQ) | 109 |

Erstaunlich zeigte sich auch hier, dass antimikrobiell bekannte Stoffe (Alexidine) keinerlei desodorierende Wirksamkeit aufweisen.

Erstaunlich ist jedoch vor allem, dass Polyquaternium Polymere ausgewählt aus der Gruppe Polyquaternium-16, Polyquaternium-6 und/oder Polyquaternium-37, und vor allem PQ-16, auf Textilien aufgebracht zu einer beachtenswerten Deowirkung führen.

Es zeigte sich, dass insbesondere Polyquaternium-16 Polymere mit einem VP/QVI Verhältnis im Bereich von 5 zu 95 (Luviquat Excellence) eine besonders gute desodorierende Wirkung zeigen (Reduktionsfaktor von 1335).

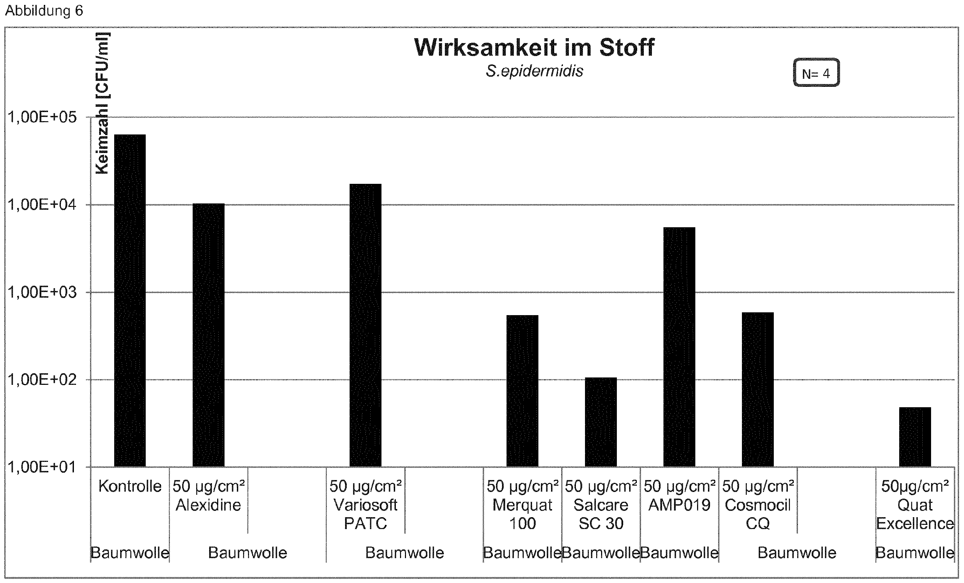

Die Keimzahlreduktion unterschiedlicher antimikrobieller Stoffe auf Baumwolle zeigt Abbildung 6. Die angegebenen Konzentrationswerte beziehen sich auf Gewicht pro Quadratzentimeter an reinem Wirkstoff (Aktivmenge).

Es wurden getestet ein Verdicker Palmitamidopropyltrimonium Chloride (Variosoft PATC), Polyaminopropyl Biguanide (Cosmocil CQ), Polyquaternium-6 (Merquat 100, Salcare SC 30), Polyquaternium 37 (AMP019) und PQ-16 (Luviquat Excellence).

Alle getesteten Polyquaternium Polymere zeigen gegenüber den Vergleichszubereitungen Alexidine und Palmitamidopropyltrimonium Chloride eine signifikant bessere Keimreduktion. Auch gegen Polyaminopropyl Biguanide zeigen PQ-6 und PQ-16 verbesserte Keimreduktionswerte.

Der Unterschied der Werte Merquat 100 zu Salcare SC 30, obwohl beide Handelsprodukte PQ-6 umfassen, liegt in der unterschiedlichen Herstellweise und dem damit verbundenen unterschiedlichen Restmonomergehalt.

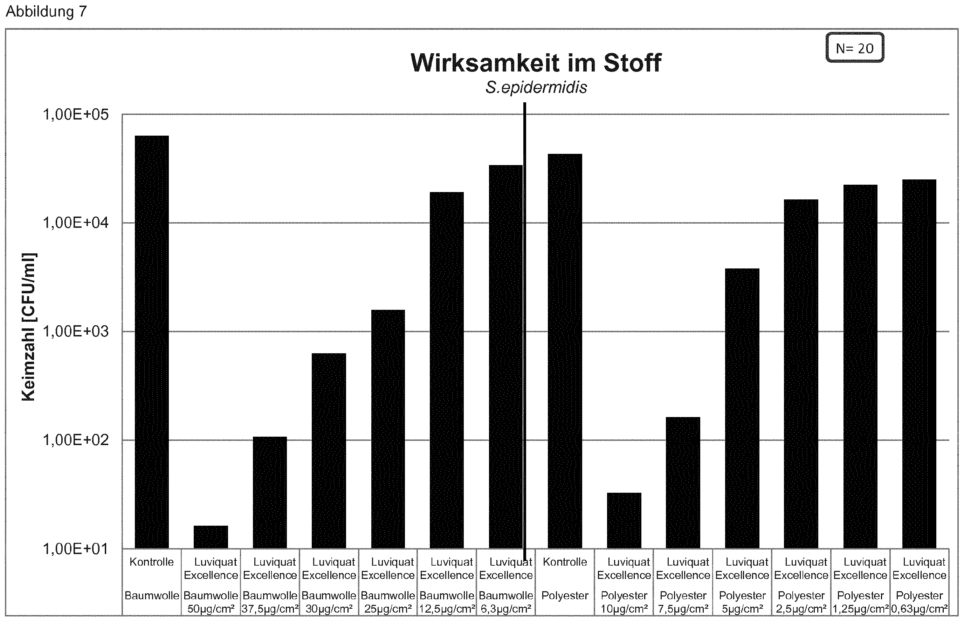

In einer weiteren Untersuchung wurde der Einfluss der Konzentration der Polyquaternium Polymere auf die Deowirkung auf Textilien untersucht (Abbildung 7).

Es wurde dazu wie zuvor dargestellt die Keimzahlreduktion unterschiedlich konzentrierter PQ-16 Polymeren (Luviquat Excellence) auf Baumwolle und Polyester Textilien ermittelt. Die angegebenen Konzentrationswerte beziehen sich auf Gewicht pro Quadratzentimeter an reinem Wirkstoff (PQ-16).

In den Untersuchungen zeigten sich auch untere Wirkgrenzen, die bei Aufbringen auf Baumwolle bei $12{,}5\,\mu g/cm^2$ und bei Polyester bei $2{,}5\,\mu g/cm^2$ liegen. Unterhalb dieser Auftragungsmenge ist keine Deowirkung mehr zu beobachten.

In einem weiteren Sniff-Test wurde die Deowirksamkeit von PQ-16 auf Textilien untersucht.

Hierzu wurden Baumwoll-Pads mit einer Konzentration von $5\,mg/cm^2$ Tränkungsmedien getränkt, 14h getrocknet, in die T-Shirts geklettet und 8h getragen.

Die getesteten Tränkungsmedien umfassten einmal Verum bestehend aus 3,75% Luviquat Excellence (40%), 45 % Ethanol, 51,25% Wasser und zum anderen das Kontrollmedium (Vehikel) bestehend aus 45% Ethanol und 55% Wasser.

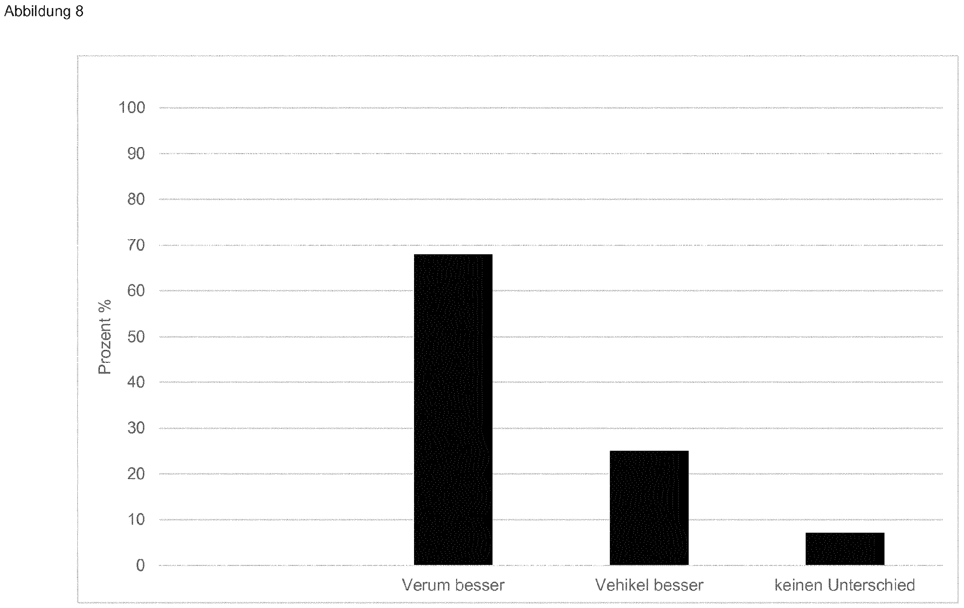

Entsprechend den oben ausgeführten Sniff-Test Bedingungen ergaben sich folgende Resultate wie sie in Abbildung 8 dargestellt sind.

Nach 8h konnte bei 67,9% der Probanden in der mit der erfindungsgemäßen Zubereitung behandelten Achsel eine signifikante Reduktion des Schweißgeruchs durch das Sniffer-Panel wahrgenommen werden. Bei 7,1% der Probanden konnte kein Unterschied festgestellt werden. Bei 25% der Probanden wurde die unbehandelte Achsel besser bewertet.

Polyquaternium Polymeren ausgewählt aus der Gruppe Polyquaternium-16, Polyquaternium-6 und/oder Polyquaternium-37 sind demnach für die Desodorierung von Textilien geeignet.

In der Anwendung der Zubereitungen mit PQ-Polymeren auf oder durch die Kleidung bieten sich vorzugsweise Polyquaternium Polymere enthaltene Zubereitungen an, die als Aerosol- oder Pumpspray konzipiert sind.

Verwendete Zubereitungen sind bevorzugt O/W-Emulsionen, Makroemulsionen, Mikroemulsionen, alkoholische Zubereitungen, PIT-Emulsionen und Hydrodispersionen.

Die kosmetischen oder dermatologischen Zubereitungen können ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Lipide, Substanzen zum Verhindern des Schäumens, Farbstoffe und Farbpigmente, Verdickungsmittel, anfeuchtende und/oder feuchthaltende Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

Die vorstehenden und nachfolgenden Beispiele veranschaulichen verwendete Zubereitungen. Die Zahlenangaben sind Gewichtsanteile bezogen auf die Gesamtasse der Zubereitung, sofern nichts anderes angegeben ist. Die Anteilsangaben beziehen sich auf die eingesetzten Rohstoffe bzw. Handelsprodukte. Der Aktivgehalt an PQ-Polymeren ergibt sich dann aus den Herstellerangaben, wie

PQ-6

- SALCARE SC 30 (39-41% PQ-6 in Wasser)
- MIRAPOL 100 (40% PQ-6 in Wasser)

PQ-16

- Luviquat Excellence (38-42% PQ-16 in Wasser)
- Luviquat FC 550 (38-42% PQ-16 in Wasser)
- Luviquat FC 370 (38-42% PQ-16 in Wasser)
- Luviquat Style (19-21% PQ-16 in Wasser)

Beispiele

1) Mikroemulsionsgele

| INCI: O/W Emulsion | 24 | 32 | 31a | 32a | 33 | 34 | 35 |
|---|---|---|---|---|---|---|---|
| Persea Gratissima Oil | 0,5 | 0,1 | 0 | 0,1 | 0 | 0,1 | 0,1 |
| Pionier 2076 (Paraffinum Liquidum; Hansen & Rosenthal) | 4 | 3 | 0 | 3 | 2,8 | 0 | 3 |
| Tegin ISO (Glyceryl Isostearate; Evonik Industries) | 2,2 | 2,2 | 2,4 | 2,4 | 1,5 | 2,1 | 2,2 |
| Tego Alkanol IC 20 (Isoceteth-20; Evonik Industries) | 4,8 | 5 | 4,8 | 4,7 | 3,65 | 5 | 5 |
| Cetiol OE (Dicaprylyl Ether, BASF Personal Care and Nutrition) | 0 | 0 | 3 | 0 | 0 | 2 | 0 |
| Glycerin | 2 | 2 | 0 | 0 | 0 | 2 | 2 |
| Trisodium EDTA | 0,5 | 0,2 | 0,5 | 0,5 | 0,2 | 0,2 | 0,2 |
| Octopirox (Piroctone Olamine; Clariant) | 0,05 | 0,05 | 0,05 | 0,05 | 0,05 | 0,05 | 0,05 |
| Parfum | q.s. | q.s | q.s | q.s | q.s | q.s | q.s |
| Butylene Glycol | 4 | 3 | 3 | 3 | 3 | 3 | 3 |
| Kessco PEG 6000 DS HV (PEG-150 Distearate; Italmatch Chemical) | 0,7 | 0,75 | 0,7 | 0,7 | 1,2 | 0,7 | 0,85 |
| Luviquat Excellence (Polyquaternium-16; BASF) | 2,5 | 2,5 | 0 | 0 | 0 | 0 | 0 |
| Luviquat FC 550 (Polyquaternium-16; BASF) | 0 | 0 | 1,25 | 4 | 0 | 0 | 2,5 |
| Luviquat FC 370 (Polyquaternium-16; BASF) | 0 | 0 | 0 | 0 | 3,5 | 0 | 0 |
| Luviquat Style (Polyquaternium-16; BASF) | 0 | 0 | 0 | 0 | 0 | 3,5 | 0 |
| Aqua | ad 100 | | | | | | |
| Resultat: Homogene Zubereitung | | | | | | | |

2) Makroemulsionen

| INCI O/W Emulsion | 36 | 37 | 38 | 39 | 40 | 41 |
|---|---|---|---|---|---|---|
| Persea Gratissima Oil | 0,1 | 0,1 | 0,25 | 0,2 | 0,1 | 0,1 |
| Cetiol E ( PPG-15 Stearyl Ether; BASF Personal Care and Nutrition) | 4 | 4 | 3 | 4 | 2 | 2 |
| Tego Alkanol S 21 (Steareth-21; Evonik Industries) | 2,5 | 2,3 | 2,0 | 2,6 | 3 | 2,8 |

(fortgesetzt)

| INCI O/W Emulsion | 36 | 37 | 38 | 39 | 40 | 41 |
|---|---|---|---|---|---|---|
| Tego Alkanol S2 (Steareth-2; Evonik Industries) | 1,5 | 1,7 | 2,0 | 1,7 | 1 | 1,2 |
| Parfum | q.s. | q.s. | q.s. | q.s. | q.s. | q.s. |
| Trisodium EDTA | 1,5 | 1,5 | 1,5 | 1,5 | 1,5 | 1,5 |
| Piroctone Olamine (Octopirox; Clariant) | 0,05 | 0,05 | 0,05 | 0,05 | 0,05 | 0,05 |
| Luviquat Excellence (Polyquaternium-16; BASF) | 1,5 | 0 | 0 | 0 | 2 | 0 |
| Luviquat FC 550 (Polyquaternium-16; BASF) | 0 | 2 | 0 | 0 | 0 | 0 |
| Luviquat FC 370 (Polyquaternium-16; BASF) | 0 | 0 | 1 | 0 | 0 | 1,5 |
| Luviquat Style (Polyquaternium-16; BASF) | 0 | 0 | 0 | 1,5 | 0 | 0 |
| Aqua | ad 100 | | | | | |
| Resultat: Homogene Zubereitung | | | | | | |

### 3) Alkoholische Zubereitungen

| INCI: Alkoholische Lösung | 42 | 43 | 44 | 45 | 46 | 47 | 48 | 49 |
|---|---|---|---|---|---|---|---|---|
| Citric Acid | 0,05 | 0,1 | 0,05 | 0,1 | 0,05 | 0,1 | 0,05 | 0,1 |
| Diammonium Citrate | 0 | 0,3 | 0 | 0 | 0 | 0,3 | 0 | 0 |
| Natrosol 250 HHX pharm (Hydroxyethylcellulose; Ashland Aqualon Functional Ingredients) | 0,2 | 0 | 0,3 | 0 | 0,35 | 0 | 0,2 | 0 |
| Eutanol G (Octyldodecanol; BASF Personal Care and Nutrition) | 0,1 | 0,1 | 0,1 | 0,1 | 0,1 | 0,1 | 0,1 | 0,1 |
| Ethanol | 30 | 45 | 30 | 45 | 30 | 45 | 30 | 45 |
| Parfum | q.s. | q.s. | q.s. | q.s. | q.s. | q.s. | q.s. | q.s. |
| Polyglykol 400 (PEG-8; Clariant) | 1,0 | 0 | 1,0 | 0 | 1,0 | 0 | 1,0 | 0 |
| Cremophor RH 410 ( PEG-40 Hydrogenated Castor Oil; BASF) | 1,5 | 1 | 1,5 | 1 | 1,5 | 1 | 1,5 | 1 |
| Luviquat Excellence (Polyquaternium-16; BASF) | 4 | 3,5 | 0 | 0 | 0 | 0 | 0 | 0 |
| Luviquat FC 550 (Polyquaternium-16; BASF) | 0 | 0 | 3 | 2,5 | 0 | 0 | 0 | 0 |
| Luviquat FC 370 (Polyquaternium-16; BASF) | 0 | 0 | 0 | 0 | 3,5 | 3 | 0 | 0 |
| Luviquat Style (Polyquaternium-16; BASF) | 0 | 0 | 0 | 0 | 0 | 0 | 2 | 3 |
| Aqua | Ad 100 | | | | | | | |
| Resultat: Homogene Zubereitung | | | | | | | | |

### 4) transparente und translucente PIT-Emulsionen

| INCI | 50 | 51 | 52 | 53 |
|---|---|---|---|---|
| Eutanol G (Octyldodecanol; BASF Personal Care and Nutrition) | 3 | 0,1 | 0 | 3 |
| Cetiol CC (Dicaprylyl Carbonate; BASF Personal Care and Nutrition) | 1 | 0 | 0 | 1 |

(fortgesetzt)

| INCI | 50 | 51 | 52 | 53 |
|---|---|---|---|---|
| Cetiol OE (Dicaprylyl Ether, BASF Personal Care and Nutrition) | 0 | 5 | 3 | 0 |
| Cetiol LC (Coco-Caprylate/Caprate, BASF Personal Care and Nutrition) | 0 | 4,5 | 3 | 0 |
| Luviquat Excellence (Polyquaternium-16; BASF) | 0 | 2,5 | 0 | 0 |
| Luviquat FC 550 (Polyquaternium-16; BASF) | 1,5 | 0 | 0 | 0 |
| Luviquat FC 370 (Polyquaternium-16; BASF) | 0 | 0 | 2 | 0 |
| Luviquat Style (Polyquaternium-16; BASF) | 0 | 0 | 0 | 1 |
| Brij IC20N-SO-(AP) (Isoceteth-20; Croda) | 0 | 0 | 4,7 | 0 |
| Tegin ISO Spezial (Glyceryl Isostearate; Evonik Industries) | 2 | 0 | 2,50 | 1,8 |
| Rewoderm 66 E 20 (Isosteareth-20; Evonik Industries) | 4 | 0 | 0 | 5,2 |
| Eumulgin B 2 (Ceteareth-20; BASF Personal Care and Nutrition) | 0 | 2 | 2,1 | 0 |
| Emulgade SE-PF (Glyceryl Stearate + Ceteareth-20 + Cetearyl Alcohol + Cetyl Palmitate + Ceteareth-12; BASF Personal Care and Nutrition) | 0 | 3,8 | 3,7 | 0 |
| Glycerin | 3 | 2,6 | 2,2 | 3 |
| Piroctone Olamine (Octopirox; Clariant) | 0,05 | 0,05 | 0,05 | 0,05 |
| Butylene Glycol | 0 | 0 | 3 | 0 |
| Trisodium EDTA | 0,5 | 0,2 | 0,3 | 0,2 |
| Aqua | ad 100 | | | |
| Parfum | q.s. | q.s. | q.s. | q.s |
| Resultat | Homogene Zubereitung | | | |

5. O/W Emulsionen

| INCI: O/W-Emulsion | 54 | 55 | 56 | 57 |
|---|---|---|---|---|
| Varisoft TA 100 (Distearyldimonium Chloride; Evonik Industries) | 1 | 1,25 | 1 | 1,25 |
| Glycerin | 25 | 30 | 28 | 27 |
| Lipocire NA-10/B Pastillen (Hydrogenated Coco-Glycerides; Gattefosse) | 2,5 | 2 | 2,5 | 2 |
| Stearyl Alcohol | 2 | 2 | 2 | 2 |
| Luviquat Excellence (Polyquaternium-16; BASF) | 2,5 | 0 | 0 | 0 |
| Luviquat FC 550 (Polyquaternium-16; BASF) | 0 | 2 | 0 | 0 |
| Luviquat FC 370 (Polyquaternium-16; BASF) | 0 | 0 | 1,75 | 0 |
| Luviquat Style (Polyquaternium-16; BASF) | 0 | 0 | 0 | 2,0 |
| Parfum | q.s. | q.s. | q.s. | q.s. |
| Octopirox (Piroctone Olamine; Clariant) | 0,05 | 0,05 | 0,05 | 0,05 |
| Aqua | Ad 100 | | | |
| Resultat: homogene Zubereitung | | | | |

**Patentansprüche**

1. Verwendung von Polyquaternium Polymeren ausgewählt aus der Gruppe Polyquaternium-16-, Polyquaternium-6- und/oder Polyquaternium-37 Polymere als antimikrobieller Deodorantwirkstoff in kosmetischen oder dermatologischen Zubereitungen.

2. Verwendung nach Anspruch 1, **dadurch gekennzeichnet, dass** als Polyquaternium Polymere Polyquaternium-16 Polymere (3-Methyl-1-Vinylimidazolium Chlorid-1-Vinyl-2-Pyrrolidinon Chloride) gewählt werden.

3. Verwendung nach Anspruch 2, **dadurch gekennzeichnet, dass** die Polyquaternium-16 Polymere (3-Methyl-1-Vinylimidazolium Chlorid-1-Vinyl-2-Pyrrolidinon Chloride) gewählt werden aus der Gruppe, die

   - gebildet sind aus einem Monomerverhältnis von Vinylpyrrolidon (VP) zu quaternisiertem Vinylimidazol (QVI) im Bereich von 1 bis 99% (VP) zu 99 bis 1% (QVI), insbesondere im Bereich von 5 bis 95% (VP/QVI),
   - eine Molmasse im Bereich von 10.000 bis 1.000.000 Da und
   - eine Ladungsdichte im Bereich von 1 bis 7 meq/g aufweisen.

4. Verwendung nach einem der vorstehenden Ansprüche, **dadurch gekennzeichnet, dass** die Polyquaternium Polymere in einer kosmetischen oder dermatologischen Zubereitung enthalten sind, die neben dem oder den Polyquaternium Polymeren frei von antitranspirant wirkenden Stoffen ist.

5. Verwendung nach einem der vorstehenden Ansprüche 3 bis 4, **dadurch gekennzeichnet, dass** der Anteil an einem oder mehreren Polyquaternium Polymeren bis zu 10 Gew.%, insbesondere bis zu 2 Gew.%, bezogen auf die Gesamtmasse der Zubereitung gewählt wird.

**Claims**

1. Use of polyquaternium polymers selected from the group consisting of polyquaternium-16, polyquaternium-6 and/or polyquaternium-37 polymers as antimicrobial deodorant active ingredient in cosmetic or dermatological preparations.

2. Use according to Claim 1, **characterized in that** the polyquaternium polymers selected are polyquaternium-16 polymers (3-methyl-1-vinylimidazolium chloride-1-vinyl-2-pyrrolidinone chloride).

3. Use according to Claim 2, **characterized in that** the polyquaternium-16 polymers (3-methyl-1-vinylimidazolium chloride-1-vinyl-2-pyrrolidinone chloride) are selected from the group

   - formed from a monomer ratio of vinyl pyrrolidone (VP) to quaternized vinylimidazole (QVI) in the range of 1 to 99% (VP) to 99 to 1% (QVI), especially in the range of 5 to 95% (VP/QVI),
   - having a molar mass in the range of 10 000 to 1 000 000 Da and
   - a charge density in the range of 1 to 7 meq/g.

4. Use according to any of the preceding claims, **characterized in that** the polyquaternium polymers are present in a cosmetic or dermatological preparation which, besides the polyquaternium polymer(s), is free from antiperspirant substances.

5. Use according to either of the preceding Claims 3 to 4, **characterized in that** the proportion of one or more polyquaternium polymers is selected up to 10% by weight, especially up to 2% by weight, based on the total mass of the preparation.

**Revendications**

1. Utilisation de polymères de type polyquaternium choisis dans le groupe des polymères de type polyquaternium-16, polyquaternium-6 et/ou polyquaternium-37 en tant que principe actif déodorant antimicrobien dans des préparations cosmétiques ou dermato-logiques.

**2.** Utilisation selon la revendication 1, **caractérisée en ce qu'**en tant que polymères de type polyquaternium des polymères de type polyquaternium-16 (chlorures de chlorure de 3-méthyl-1-vinylimidazolium-1-vinyle-2-pyrrolidone) sont choisis.

**3.** Utilisation selon la revendication 2, **caractérisée en ce que** les polymères de type polyquaternium-16 (chlorures de chlorure de 3-méthyl-1-vinylimidazolium-1-vinyle-2-pyrrolidone) sont choisis dans le groupe de ceux qui

- sont formés à partir d'un rapport de monomère de vinylpyrrolidone (VP) sur vinyli-midazole quaternisé (QVI) dans la plage de 1 à 99 % (VP) sur 99 à 1 % (QVI), en particulier dans la plage de 5 à 95 % (VP/QVI),
- présentent une masse molaire dans la plage de 10 000 à 1 000 000 Da et
- présentent une densité de charge dans la plage de 1 à 7 méq/g.

**4.** Utilisation selon l'une quelconque des revendications précédentes, **caractérisée en ce que** les polymères de type polyquaternium sont contenus dans une préparation cosmétique ou dermatologique, qui, outre le ou les polymères de type polyquaternium, est exempte de matières à effet antitranspirant.

**5.** Utilisation selon l'une quelconque des revendications précédentes 3 et 4, **caractérisée en ce que** la proportion d'un ou plusieurs polymères de type polyquaternium est choisie jusqu'à 10 % en poids, en particulier jusqu'à 2 % en poids, par rapport à la masse totale de la préparation.

Abbildung 1

EP 3 171 944 B1

Abbildung 2

**Sniff-Test**

%

| | nach 24 h | | | nach 48 h | |
kein Unterschied / unbehandelt / Beispiel 24 (für beide Zeiträume)

Abbildung 3

Abbildung 4

nach 48 h

Abbildung 5

EP 3 171 944 B1

Abbildung 6

Wirksamkeit im Stoff
*S.epidermidis*

N= 4

Keimzahl [CFU/ml]

y-axis: 1,00E+05, 1,00E+04, 1,00E+03, 1,00E+02, 1,00E+01

Kontrolle — Baumwolle
50 µg/cm² Alexidine — Baumwolle
50 µg/cm² Variosoft PATC — Baumwolle
50 µg/cm² Merquat 100 — Baumwolle
50 µg/cm² Salcare SC 30 — Baumwolle
50 µg/cm² AMP019 — Baumwolle
50 µg/cm² Cosmocil CQ — Baumwolle
50µg/cm² Quat Excellence — Baumwolle

Abbildung 7

**Wirksamkeit im Stoff**

*S.epidermidis*

N= 20

EP 3 171 944 B1

Abbildung 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013052454 A1
- EP 1504744 B1
- US 20070184016 A1
- WO 2007095008 A2
- EP 1991654 A1
- EP 200548 A2
- WO 199524105 A1
- US 4743440 A
- WO 2003030853 A2
- WO 2009091794 A1
- EP 1761241 A1
- JP 2014101356 B
- WO 1998015254 A1

**In der Beschreibung aufgeführte Nicht-Patentliteratur**

- **ZEITSCHRIFT FÜR FETT ; ÖL ; KOSMETIK.** *Pharma- und Waschmittelindustrie,* Marz 1985
- *J. Soc. Cosmet. Chem.,* Juni 1987, vol. 38, 223-231